# EUROPEAN PATENT APPLICATION

(11) **EP 2 749 643 A1**
(43) Date of publication of application: **02.07.2014**
(21) Application number: 12306718.3
(22) Date of filing: 31.12.2012
(51) Int. Cl.: C12N 9/00

(54) **Novel bacterial iodoperoxidases from Zobellia galactanivorans, methods of preparation and uses thereof**

(71) Applicant: Centre National de la Recherche Scientifique (CNRS), 75016 Paris (FR); Université Pierre et Marie Curie (Paris 6), 75005 Paris (FR)
(72) Inventor: Leblanc, Catherine, 29640 Plougonven (FR); Delage, Ludovic, 29420 Mespaul (FR); Fournier, Jean-Baptiste, 29660 Carantec (FR); Michel, Gurvan, 29250 Plougoulm (FR); Potin, Philippe, 29680 Roscoff (FR); Czjzek, Mirjam, 29250 Plougoulm (FR)
(74) Representative: Regimbeau

(57) **Abstract**

The present invention concerns novel iodoperoxidases from *Zobellia galactanivorans,* isolated nucleic acids encoding same, as well as methods for preparing these enzymes.

Moreover, the invention is also directed to the use of such iodoperoxidases in a wide range of industrial, pharmaceutical, medical, cosmetics, and ecological applications, as well as in the food industry.

SEQUENCE LISTING

<110>  CENTRE NATIONAL DE LA RECHERCHE SCIENTIFIQUE (CNRS)
       UNIVERSITE PIERRE ET MARIE CURIE (PARIS 6)

<120>  NOVEL BACTERIAL IODOPEROXIDASES FROM Zobellia galactanivorans,
       METHODS OF PREPARATION AND USES THEREOF

<130>  70382D31841

<160>  54

<170>  PatentIn version 3.5

<210>  1
<211>  414
<212>  PRT
<213>  artificial

<220>
<223>  ZgvIPO2

<400>  1

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr

165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  2
<211>  1242

<212>  DNA
<213>  artificial

<220>
<223>  ZgvIPO2

<400>  2
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242


<210>  3
<211>  441
<212>  PRT
<213>  Zobellia galactanivorans


<220>
<221>  misc_feature
<223>  ZgvIPO2 with the putative native peptide signal (wild-type
       ZgvIPO2)

<400>  3

Met Lys Arg Leu Ser Arg Phe Ile Ile Val Cys Thr Val Leu Met Val
1               5                   10                  15

Ser Cys Gly Thr Asn His Asp Lys Glu Arg Leu Asp Thr Tyr Phe Glu
            20                  25                  30

Gly Gly Leu Ser Arg Tyr Asn Arg Glu Leu Thr Asn Val Ile Val Ser
        35                  40                  45

Asp Ile Phe Thr Pro Pro Val Ala Ser Arg Ile Tyr Ala Tyr Ser Asn
    50                  55                  60

Ile Ala Ala Tyr Glu Gly Ile Arg Phe Met Asp Ser Thr Lys Leu Ser
65                  70                  75                  80

Leu Ser Ser Arg Leu Asn Gly Leu Lys Glu Leu Ser Thr Pro Asp Pro
                85                  90                  95

Ser Lys Asn Tyr Tyr Tyr Pro Leu Val Ser Ile Val Ala Phe Thr Gln
            100                 105                 110

Val Gly Lys Ser Leu Val Phe Asp Leu Asp Lys Val Glu Ala Ile Lys
        115                 120                 125

Asn Lys Met Leu Gly Glu Val Lys Glu Ile Gly Met Asp Gln Glu Val
    130                 135                 140

Tyr Arg Asn Ser Ile Asp Leu Gly Glu Thr Leu Ala Ser Glu Ile Leu
145                 150                 155                 160

Ala Trp Ala Ala Lys Asp Gly Tyr Leu Arg Arg Thr Ala Leu Pro Arg
                165                 170                 175

Tyr Ser Val Ser Asp Asp Pro Gly Arg Trp Arg Pro Thr Pro Pro Asp
            180                 185                 190

Tyr Met Glu Ala Ile Glu Pro His Trp Asn Thr Leu Arg Pro Phe Val
        195                 200                 205

Leu Asp Ser Ala Ala Gln Phe Asp Pro Gly Leu Pro Thr Pro Phe Asp
    210                 215                 220

Ser Ala Glu Asp Ser Pro Phe Tyr Lys Glu Ala Met Glu Val Tyr Glu
225                 230                 235                 240

Thr Val Glu Asn Leu Asp Asp Asp Lys Leu Glu Val Ala Lys Phe Trp
                245                 250                 255

Asp Cys Asn Pro Asn Ile Ser His Thr Lys Gly His Val Met Tyr Phe
            260                 265                 270

Gln Gln Gln Ile Ser Pro Gly Gly His Trp Met His Ile Ala Ala Gln
        275                 280                 285

Ile Leu Glu Glu Gln Lys Val Asp Gly Val Lys Ala Ala Glu Thr Met
    290                 295                 300

Ser Met Leu Gly Val Ala Leu Ala Asp Ala Phe Ile Ser Cys Trp Asp
305                 310                 315                 320

Gln Lys Tyr Lys Ser Ser Leu Thr Arg Pro Glu Thr Tyr Ile Asn Asn
                325                 330                 335

Tyr Ile Asp Gln Asp Trp Glu Pro Ile Leu Gln Thr Pro Ala Phe Pro
            340                 345                 350

Glu His Thr Ser Gly His Ser Val Ala Ser Ser Ala Ala Ala Thr Val
        355                 360                 365

Leu Thr Gly Val Phe Gly Asp Asp Tyr Ala Phe Val Asp Ala Thr Glu
    370                 375                 380

Val Pro Tyr Gly Leu Pro Pro Arg Ser Phe Gln Ser Phe Trp Gln Ala
385                 390                 395                 400

Ala Glu Glu Ala Ala Ile Ser Arg Leu Tyr Gly Gly Ile His Tyr Arg
                405                 410                 415

Pro Ala Ile Glu Leu Gly Val Lys Gln Gly Arg Ala Val Gly Arg Leu
            420                 425                 430

Val Leu Glu Arg Leu Arg Lys Glu His
        435                 440

<210>  4
<211>  1326
<212>  DNA
<213>  Zobellia galactanivorans

<220>
<221>  misc_feature
<223>  ZgvIPO2 with the putative native peptide signal (wild-type
       ZgvIPO2)

<400>  4
atgaagcgat taagtaggtt tattatcgta tgtacggtgc ttatggtatc ctgtggaaca      60

aatcacgaca aagagcggtt agatacgtat tttgaaggcg gtttgtcgag atacaaccgg     120

gaactcacca atgtaatcgt atctgatatt tttaccccac cggtggccag taggatctat     180

gcttattcca atattgcggc ctacgaaggg atacgcttta tggactcgac aaaattgtcc     240

ctgtcgtccc gattgaatgg gttaaaagag ttatcgacac ctgatccgtc caagaattat 300

tactatccct tagtgtcaat tgtagcgttt acccaggtgg gaaaatcctt ggtttttgat 360

ttggacaagg tcgaggccat caagaacaag atgctcgggg aggtcaagga aataggtatg 420

gaccaagaag tttatcggaa ttcaattgat ctaggggaga ccctggcaag cgaaatcttg 480

gcttgggcgg caaaagatgg gtatctccgc cgtacggcat tgccccgata ttcggtcagt 540

gatgacccgg gccgttggag gcctacgcca ccggactata tggaggccat tgaaccgcat 600

tggaatacct tacggccctt tgtgcttgat tcggcagcac aatttgatcc aggactgccc 660

acgccttttg acagtgccga agactcccca ttttataagg aagccatgga ggtctatgaa 720

acggtggaaa atctagatga tgataaattg gaggtggcga agttttggga ctgtaacccg 780

aatatatcgc acaccaaggg gcatgtcatg tatttccaac aacaaatttc ccctgggggc 840

cattggatgc atatagctgc acagatactg gaagaacaaa aggtcgacgg ggtaaaggct 900

gccgaaacca tgtcgatgtt aggggtggcc ctggccgatg cctttatcag ttgttgggac 960

cagaagtata aaagcagttt gacccggcct gaaacctata tcaataacta tattgaccaa 1020

gattgggaac ctatattgca aacgccggct tttcctgagc atacgtctgg acatagtgtg 1080

gcgtcaagtg cagcagctac ggtacttacc ggggtattcg gcgatgatta tgcatttgta 1140

gatgctacgg aagtaccata cggccttcct ccaagaagct ttcagtcgtt ttggcaagca 1200

gcggaagaag cagcgatcag caggctttac ggggggattc actataggcc ggccattgaa 1260

ctgggcgtta aacagggcag ggccgtgggc cgattggttc tcgagcgatt aagaaaggag 1320

cattga 1326

<210> 5
<211> 428
<212> PRT
<213> artificial

<220>
<223> ZgvIPO2 with a histidine tag

<400> 5

Met Arg Gly Ser His His His His His His Gly Ser Ala Cys Asp Thr
1               5                   10                  15

Tyr Phe Glu Gly Gly Leu Ser Arg Tyr Asn Arg Glu Leu Thr Asn Val
            20                  25                  30

Ile Val Ser Asp Ile Phe Thr Pro Pro Val Ala Ser Arg Ile Tyr Ala
        35                  40                  45

Tyr Ser Asn Ile Ala Ala Tyr Glu Gly Ile Arg Phe Met Asp Ser Thr
    50                  55                  60

Lys Leu Ser Leu Ser Ser Arg Leu Asn Gly Leu Lys Glu Leu Ser Thr
65                  70                  75                  80

Pro Asp Pro Ser Lys Asn Tyr Tyr Tyr Pro Leu Val Ser Ile Val Ala
                85                  90                  95

Phe Thr Gln Val Gly Lys Ser Leu Val Phe Asp Leu Asp Lys Val Glu
            100                 105                 110

Ala Ile Lys Asn Lys Met Leu Gly Glu Val Lys Glu Ile Gly Met Asp
        115                 120                 125

Gln Glu Val Tyr Arg Asn Ser Ile Asp Leu Gly Glu Thr Leu Ala Ser
    130                 135                 140

Glu Ile Leu Ala Trp Ala Ala Lys Asp Gly Tyr Leu Arg Arg Thr Ala
145                 150                 155                 160

Leu Pro Arg Tyr Ser Val Ser Asp Asp Pro Gly Arg Trp Arg Pro Thr
                165                 170                 175

Pro Pro Asp Tyr Met Glu Ala Ile Glu Pro His Trp Asn Thr Leu Arg
            180                 185                 190

Pro Phe Val Leu Asp Ser Ala Ala Gln Phe Asp Pro Gly Leu Pro Thr
        195                 200                 205

Pro Phe Asp Ser Ala Glu Asp Ser Pro Phe Tyr Lys Glu Ala Met Glu
    210                 215                 220

Val Tyr Glu Thr Val Glu Asn Leu Asp Asp Asp Lys Leu Glu Val Ala
225                 230                 235                 240

Lys Phe Trp Asp Cys Asn Pro Asn Ile Ser His Thr Lys Gly His Val
                245                 250                 255

Met Tyr Phe Gln Gln Gln Ile Ser Pro Gly Gly His Trp Met His Ile
            260                 265                 270

Ala Ala Gln Ile Leu Glu Glu Gln Lys Val Asp Gly Val Lys Ala Ala
        275                 280                 285

Glu Thr Met Ser Met Leu Gly Val Ala Leu Ala Asp Ala Phe Ile Ser
    290                 295                 300

Cys Trp Asp Gln Lys Tyr Lys Ser Ser Leu Thr Arg Pro Glu Thr Tyr
305                 310                 315                 320

Ile Asn Asn Tyr Ile Asp Gln Asp Trp Glu Pro Ile Leu Gln Thr Pro

325                 330                 335

Ala Phe Pro Glu His Thr Ser Gly His Ser Val Ala Ser Ser Ala Ala
            340                 345                 350

Ala Thr Val Leu Thr Gly Val Phe Gly Asp Asp Tyr Ala Phe Val Asp
        355                 360                 365

Ala Thr Glu Val Pro Tyr Gly Leu Pro Pro Arg Ser Phe Gln Ser Phe
    370                 375                 380

Trp Gln Ala Ala Glu Glu Ala Ala Ile Ser Arg Leu Tyr Gly Gly Ile
385                 390                 395                 400

His Tyr Arg Pro Ala Ile Glu Leu Gly Val Lys Gln Gly Arg Ala Val
                405                 410                 415

Gly Arg Leu Val Leu Glu Arg Leu Arg Lys Glu His
            420                 425

<210>  6
<211>  1287
<212>  DNA
<213>  artificial

<220>
<223>  ZgvIPO2  with a histidine tag

<400>  6
atgagaggat cgcatcacca tcaccatcac ggatccgcat gcgatacgta ttttgaaggc      60

ggtttgtcga gatacaaccg ggaactcacc aatgtaatcg tatctgatat ttttacccca     120

ccggtggcca gtaggatcta tgcttattcc aatattgcgg cctacgaagg gatacgcttt     180

atggactcga caaaattgtc cctgtcgtcc cgattgaatg ggttaaaaga gttatcgaca     240

cctgatccgt ccaagaatta ttactatccc ttagtgtcaa ttgtagcgtt tacccaggtg     300

ggaaaatcct tggtttttga tttggacaag gtcgaggcca tcaagaacaa gatgctcggg     360

gaggtcaagg aaataggtat ggaccaagaa gtttatcgga attcaattga tctaggggag     420

accctggcaa gcgaaatctt ggcttgggcg gcaaaagatg ggtatctccg ccgtacggca     480

ttgccccgat attcggtcag tgatgacccg ggccgttgga ggcctacgcc accggactat     540

atggaggcca ttgaaccgca ttggaatacc ttacggccct ttgtgcttga ttcggcagca     600

caatttgatc caggactgcc cacgcctttt gacagtgccg aagactcccc attttataag      660

gaagccatgg aggtctatga aacggtggaa aatctagatg atgataaatt ggaggtggcg     720

aagttttggg actgtaaccc gaatatatcg cacaccaagg ggcatgtcat gtatttccaa     780

caacaaattt cccctggggg ccattggatg catatagctg cacagatact ggaagaacaa     840

aaggtcgacg gggtaaaggc tgccgaaacc atgtcgatgt taggggtggc cctggccgat     900

gcctttatca gttgttggga ccagaagtat aaaagcagtt tgacccggcc tgaaacctat 960

atcaataact atattgacca agattgggaa cctatattgc aaacgccggc ttttcctgag 1020

catacgtctg gacatagtgt ggcgtcaagt gcagcagcta cggtacttac cggggtattc 1080

ggcgatgatt atgcatttgt agatgctacg gaagtaccat acggccttcc tccaagaagc 1140

tttcagtcgt tttggcaagc agcggaagaa gcagcgatca gcaggcttta cggggggatt 1200

cactataggc cggccattga actgggcgtt aaacagggca gggccgtggg ccgattggtt 1260

ctcgagcgat taagaaagga gcattga 1287

<210> 7
<211> 27
<212> PRT
<213> Zobellia galactanivorans

<220>
<221> misc_feature
<223> putative native signal peptide of the wild-type ZgvIPO2

<400> 7

Met Lys Arg Leu Ser Arg Phe Ile Ile Val Cys Thr Val Leu Met Val
1               5                   10                  15

Ser Cys Gly Thr Asn His Asp Lys Glu Arg Leu
            20                  25

<210> 8
<211> 81
<212> DNA
<213> zobellia galactanivorans

<220>
<221> misc_feature
<223> putative native signal peptide of the wild type ZgvIPO2

<400> 8
atgaagcgat taagtaggtt tattatcgta tgtacggtgc ttatggtatc ctgtggaaca 60

aatcacgaca aagagcggtt a 81

<210> 9
<211> 14
<212> PRT
<213> artificial

<220>
<223> histidine tag

<400> 9

Met Arg Gly Ser His His His His His His Gly Ser Ala Cys
1               5                   10

<210>  10
<211>  42
<212>  DNA
<213>  artificial

<220>
<223>   histidine tag

<400>  10
atgagaggat cgcatcacca tcaccatcac ggatccgcat gc                          42


<210>  11
<211>  46
<212>  DNA
<213>  artificial

<220>
<223>  forward primer

<400>  11
accatcacgg atccgcatgc gatacgtatt ttgaaggcgg tttgtc                      46


<210>  12
<211>  45
<212>  DNA
<213>  artificial

<220>
<223>  reverse primer

<400>  12
gcaggtcgac ccggggtacc tcaatgctcc tttcttaatc gctcg                       45


<210>  13
<211>  414
<212>  PRT
<213>  artificial

<220>
<223>  consensus sequence of a ZgvIPO2 functional variant


<220>
<221>  misc_feature
<222>  (234)..(234)
<223>  Xaa can be any naturally occurring amino acid

<220>
<221>  misc_feature
<222>  (237)..(237)
<223>  Xaa can be any naturally occurring amino acid

<220>
<221>  misc_feature
<222>  (244)..(244)
<223>  Xaa can be any naturally occurring amino acid

<220>
<221>  misc_feature
<222>  (291)..(293)
<223>  Xaa can be any naturally occurring amino acid

<220>
<221>  misc_feature
<222>  (295)..(295)
<223>  Xaa can be any naturally occurring amino acid

<220>
<221>  misc_feature
<222>  (302)..(302)
<223>  Xaa can be any naturally occurring amino acid

<220>
<221>  misc_feature
<222>  (324)..(324)
<223>  Xaa can be any naturally occurring amino acid

<220>
<221>  misc_feature
<222>  (329)..(330)
<223>  Xaa can be any naturally occurring amino acid

<220>
<221>  misc_feature
<222>  (381)..(381)
<223>  Xaa can be any naturally occurring amino acid

<400>  13

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg

145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Xaa Ile Ser Xaa Thr Lys Gly
225                 230                 235                 240

His Val Met Xaa Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Xaa Xaa Xaa Gln Xaa Tyr Lys Ser Ser Leu Thr Xaa Pro Glu
    290                 295                 300

Thr Tyr Ile Asn Asn Tyr Ile Asp Gln Asp Trp Glu Pro Ile Leu Gln
305                 310                 315                 320

Thr Pro Ala Xaa Pro Glu His Thr Xaa Xaa His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Xaa Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  14
<211>  1242
<212>  DNA
<213>  artificial

<220>
<223>  consensus sequence of ZgvIPO2 functional variant



<220>
<221>  misc_feature
<222>  (700)..(702)
<223>  n is a, c, g, or t

<220>
<221>  misc_feature
<222>  (709)..(711)
<223>  n is a, c, g, or t

<220>
<221>  misc_feature
<222>  (730)..(732)
<223>  n is a, c, g, or t

<220>
<221>  misc_feature
<222>  (871)..(879)
<223>  n is a, c, g, or t

<220>
<221>  misc_feature
<222>  (883)..(885)
<223>  n is a, c, g, or t

<220>
<221>  misc_feature
<222>  (904)..(906)
<223>  n is a, c, g, or t

<220>
<221>  misc_feature
<222>  (970)..(972)
<223>  n is a, c, g, or t

<220>
<221>  misc_feature
<222>  (985)..(990)
<223>  n is a, c, g, or t

<220>
<221>  misc_feature
<222>  (1141)..(1143)
<223>  n is a, c, g, or t

<400>  14
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc 300

aagaacaaga tgctcgggga ggtcaaggaa ataggtatgg accaagaagt ttatcggaat 360

tcaattgatc taggggagac cctggcaagc gaaatcttgg cttgggcggc aaaagatggg 420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccgn nnatatcgnn naccaagggg 720

catgtcatgn nnttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt nnnnnnnnnc agnnntataa aagcagtttg 900

accnnncctg aaacctatat caataactat attgaccaag attgggaacc tatattgcaa 960

acgccggctn nncctgagca tacgnnnnnn catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

nnnctttacg gggggattca ctataggccg gccattgaac tgggcgttaa acagggcagg 1200

gccgtgggcc gattggttct cgagcgatta agaaaggagc at 1242

<210> 15
<211> 441
<212> PRT
<213> artificial

<220>
<223> consensus sequence of a ZgvIPO2 functional variant with the wt
 ZvgIPO2 putative native peptide signal


<220>
<221> misc_feature
<222> (261)..(261)
<223> Xaa can be any naturally occurring amino acid

<220>
<221> misc_feature
<222> (264)..(264)
<223> Xaa can be any naturally occurring amino acid

<220>
<221> misc_feature
<222> (271)..(271)
<223> Xaa can be any naturally occurring amino acid

<220>
<221> misc_feature
<222> (318)..(320)
<223> Xaa can be any naturally occurring amino acid

<220>
<221>  misc_feature
<222>  (322)..(322)
<223>  Xaa can be any naturally occurring amino acid

<220>
<221>  misc_feature
<222>  (329)..(329)
<223>  Xaa can be any naturally occurring amino acid

<220>
<221>  misc_feature
<222>  (351)..(351)
<223>  Xaa can be any naturally occurring amino acid

<220>
<221>  misc_feature
<222>  (356)..(357)
<223>  Xaa can be any naturally occurring amino acid

<220>
<221>  misc_feature
<222>  (408)..(408)
<223>  Xaa can be any naturally occurring amino acid

<400>  15

Met Lys Arg Leu Ser Arg Phe Ile Ile Val Cys Thr Val Leu Met Val
1               5                   10                  15

Ser Cys Gly Thr Asn His Asp Lys Glu Arg Leu Asp Thr Tyr Phe Glu
            20                  25                  30

Gly Gly Leu Ser Arg Tyr Asn Arg Glu Leu Thr Asn Val Ile Val Ser
        35                  40                  45

Asp Ile Phe Thr Pro Pro Val Ala Ser Arg Ile Tyr Ala Tyr Ser Asn
    50                  55                  60

Ile Ala Ala Tyr Glu Gly Ile Arg Phe Met Asp Ser Thr Lys Leu Ser
65                  70                  75                  80

Leu Ser Ser Arg Leu Asn Gly Leu Lys Glu Leu Ser Thr Pro Asp Pro
                85                  90                  95

Ser Lys Asn Tyr Tyr Tyr Pro Leu Val Ser Ile Val Ala Phe Thr Gln
            100                 105                 110

Val Gly Lys Ser Leu Val Phe Asp Leu Asp Lys Val Glu Ala Ile Lys
        115                 120                 125

Asn Lys Met Leu Gly Glu Val Lys Glu Ile Gly Met Asp Gln Glu Val
    130                 135                 140

Tyr Arg Asn Ser Ile Asp Leu Gly Glu Thr Leu Ala Ser Glu Ile Leu
145                 150                 155                 160

Ala Trp Ala Ala Lys Asp Gly Tyr Leu Arg Arg Thr Ala Leu Pro Arg
                165                 170                 175

Tyr Ser Val Ser Asp Asp Pro Gly Arg Trp Arg Pro Thr Pro Pro Asp
            180                 185                 190

Tyr Met Glu Ala Ile Glu Pro His Trp Asn Thr Leu Arg Pro Phe Val
        195                 200                 205

Leu Asp Ser Ala Ala Gln Phe Asp Pro Gly Leu Pro Thr Pro Phe Asp
    210                 215                 220

Ser Ala Glu Asp Ser Pro Phe Tyr Lys Glu Ala Met Glu Val Tyr Glu
225                 230                 235                 240

Thr Val Glu Asn Leu Asp Asp Asp Lys Leu Glu Val Ala Lys Phe Trp
                245                 250                 255

Asp Cys Asn Pro Xaa Ile Ser Xaa Thr Lys Gly His Val Met Xaa Phe
            260                 265                 270

Gln Gln Gln Ile Ser Pro Gly Gly His Trp Met His Ile Ala Ala Gln
        275                 280                 285

Ile Leu Glu Glu Gln Lys Val Asp Gly Val Lys Ala Ala Glu Thr Met
    290                 295                 300

Ser Met Leu Gly Val Ala Leu Ala Asp Ala Phe Ile Ser Xaa Xaa Xaa
305                 310                 315                 320

Gln Xaa Tyr Lys Ser Ser Leu Thr Xaa Pro Glu Thr Tyr Ile Asn Asn
                325                 330                 335

Tyr Ile Asp Gln Asp Trp Glu Pro Ile Leu Gln Thr Pro Ala Xaa Pro
            340                 345                 350

Glu His Thr Xaa Xaa His Ser Val Ala Ser Ser Ala Ala Ala Thr Val
        355                 360                 365

Leu Thr Gly Val Phe Gly Asp Asp Tyr Ala Phe Val Asp Ala Thr Glu
    370                 375                 380

Val Pro Tyr Gly Leu Pro Pro Arg Ser Phe Gln Ser Phe Trp Gln Ala
385                 390                 395                 400

Ala Glu Glu Ala Ala Ile Ser Xaa Leu Tyr Gly Gly Ile His Tyr Arg
                405                 410                 415

Pro Ala Ile Glu Leu Gly Val Lys Gln Gly Arg Ala Val Gly Arg Leu
            420                 425                 430

Val Leu Glu Arg Leu Arg Lys Glu His
        435                 440

<210>  16
<211>  1326
<212>  DNA
<213>  artificial

<220>
<223>  consensus sequence of a ZgvIPO2 functional variant with the wt
       ZgvIPO2 putative native peptide signal

<220>
<221>  misc_feature
<222>  (781)..(783)
<223>  n is a, c, g, or t

<220>
<221>  misc_feature
<222>  (790)..(792)
<223>  n is a, c, g, or t

<220>
<221>  misc_feature
<222>  (811)..(813)
<223>  n is a, c, g, or t

<220>
<221>  misc_feature
<222>  (952)..(960)
<223>  n is a, c, g, or t

<220>
<221>  misc_feature
<222>  (964)..(966)
<223>  n is a, c, g, or t

<220>
<221>  misc_feature
<222>  (985)..(987)
<223>  n is a, c, g, or t

<220>
<221>  misc_feature
<222>  (1051)..(1053)
<223>  n is a, c, g, or t

<220>
<221>  misc_feature
<222>  (1066)..(1071)
<223>  n is a, c, g, or t

<220>
<221>  misc_feature
<222>  (1222)..(1224)
<223>  n is a, c, g, or t

<400>  16

atgaagcgat taagtaggtt tattatcgta tgtacggtgc ttatggtatc ctgtggaaca 60

aatcacgaca aagagcggtt agatacgtat tttgaaggcg gtttgtcgag atacaaccgg 120

gaactcacca atgtaatcgt atctgatatt tttaccccac cggtggccag taggatctat 180

gcttattcca atattgcggc ctacgaaggg atacgcttta tggactcgac aaaattgtcc 240

ctgtcgtccc gattgaatgg gttaaaagag ttatcgacac ctgatccgtc caagaattat 300

tactatccct tagtgtcaat tgtagcgttt acccaggtgg gaaaatcctt ggtttttgat 360

ttggacaagg tcgaggccat caagaacaag atgctcgggg aggtcaagga aataggtatg 420

gaccaagaag tttatcggaa ttcaattgat ctaggggaga ccctggcaag cgaaatcttg 480

gcttgggcgg caaaagatgg gtatctccgc cgtacggcat tgccccgata ttcggtcagt 540

gatgacccgg gccgttggag gcctacgcca ccggactata tggaggccat tgaaccgcat 600

tggaatacct tacggccctt tgtgcttgat tcggcagcac aatttgatcc aggactgccc 660

acgccttttg acagtgccga agactcccca ttttataagg aagccatgga ggtctatgaa 720

acggtggaaa atctagatga tgataaattg gaggtggcga agttttggga ctgtaacccg 780

nnnatatcgn nnaccaaggg gcatgtcatg nnnttccaac aacaaatttc ccctgggggc 840

cattggatgc atatagctgc acagatactg gaagaacaaa aggtcgacgg ggtaaaggct 900

gccgaaacca tgtcgatgtt aggggtggcc ctggccgatg cctttatcag tnnnnnnnnn 960

cagnnntata aaagcagttt gaccnnncct gaaacctata tcaataacta tattgaccaa 1020

gattgggaac ctatattgca aacgccggct nnncctgagc atacgnnnnn ncatagtgtg 1080

gcgtcaagtg cagcagctac ggtacttacc ggggtattcg gcgatgatta tgcatttgta 1140

gatgctacgg aagtaccata cggccttcct ccaagaagct ttcagtcgtt ttggcaagca 1200

gcggaagaag cagcgatcag cnnnctttac ggggggattc actataggcc ggccattgaa 1260

ctgggcgtta aacagggcag ggccgtgggc cgattggttc tcgagcgatt aagaaaggag 1320

cattga 1326

<210> 17
<211> 428
<212> PRT
<213> artificial

<220>
<223> consensus sequence of ZgvIPO2 functional variant with a histidine
tag

<220>
<221> misc_feature
<222> (248)..(248)
<223> Xaa can be any naturally occurring amino acid

<220>
<221> misc_feature
<222> (251)..(251)

<223>  Xaa can be any naturally occurring amino acid

<220>
<221>  misc_feature
<222>  (258)..(258)
<223>  Xaa can be any naturally occurring amino acid

<220>
<221>  misc_feature
<222>  (305)..(307)
<223>  Xaa can be any naturally occurring amino acid

<220>
<221>  misc_feature
<222>  (309)..(309)
<223>  Xaa can be any naturally occurring amino acid

<220>
<221>  misc_feature
<222>  (316)..(316)
<223>  Xaa can be any naturally occurring amino acid

<220>
<221>  misc_feature
<222>  (338)..(338)
<223>  Xaa can be any naturally occurring amino acid

<220>
<221>  misc_feature
<222>  (343)..(344)
<223>  Xaa can be any naturally occurring amino acid

<220>
<221>  misc_feature
<222>  (395)..(395)
<223>  Xaa can be any naturally occurring amino acid

<400>  17

Met Arg Gly Ser His His His His His His Gly Ser Ala Cys Asp Thr
1               5                   10                  15

Tyr Phe Glu Gly Gly Leu Ser Arg Tyr Asn Arg Glu Leu Thr Asn Val
            20                  25                  30

Ile Val Ser Asp Ile Phe Thr Pro Pro Val Ala Ser Arg Ile Tyr Ala
        35                  40                  45

Tyr Ser Asn Ile Ala Ala Tyr Glu Gly Ile Arg Phe Met Asp Ser Thr
    50                  55                  60

Lys Leu Ser Leu Ser Ser Arg Leu Asn Gly Leu Lys Glu Leu Ser Thr
65                  70                  75                  80

Pro Asp Pro Ser Lys Asn Tyr Tyr Tyr Pro Leu Val Ser Ile Val Ala
                85                  90                  95

Phe Thr Gln Val Gly Lys Ser Leu Val Phe Asp Leu Asp Lys Val Glu
            100                 105                 110

Ala Ile Lys Asn Lys Met Leu Gly Glu Val Lys Glu Ile Gly Met Asp
        115                 120                 125

Gln Glu Val Tyr Arg Asn Ser Ile Asp Leu Gly Glu Thr Leu Ala Ser
    130                 135                 140

Glu Ile Leu Ala Trp Ala Ala Lys Asp Gly Tyr Leu Arg Arg Thr Ala
145                 150                 155                 160

Leu Pro Arg Tyr Ser Val Ser Asp Asp Pro Gly Arg Trp Arg Pro Thr
                165                 170                 175

Pro Pro Asp Tyr Met Glu Ala Ile Glu Pro His Trp Asn Thr Leu Arg
            180                 185                 190

Pro Phe Val Leu Asp Ser Ala Ala Gln Phe Asp Pro Gly Leu Pro Thr
        195                 200                 205

Pro Phe Asp Ser Ala Glu Asp Ser Pro Phe Tyr Lys Glu Ala Met Glu
    210                 215                 220

Val Tyr Glu Thr Val Glu Asn Leu Asp Asp Asp Lys Leu Glu Val Ala
225                 230                 235                 240

Lys Phe Trp Asp Cys Asn Pro Xaa Ile Ser Xaa Thr Lys Gly His Val
                245                 250                 255

Met Xaa Phe Gln Gln Gln Ile Ser Pro Gly Gly His Trp Met His Ile
            260                 265                 270

Ala Ala Gln Ile Leu Glu Glu Gln Lys Val Asp Gly Val Lys Ala Ala
        275                 280                 285

Glu Thr Met Ser Met Leu Gly Val Ala Leu Ala Asp Ala Phe Ile Ser
    290                 295                 300

Xaa Xaa Xaa Gln Xaa Tyr Lys Ser Ser Leu Thr Xaa Pro Glu Thr Tyr
305                 310                 315                 320

Ile Asn Asn Tyr Ile Asp Gln Asp Trp Glu Pro Ile Leu Gln Thr Pro
                325                 330                 335

Ala Xaa Pro Glu His Thr Xaa Xaa His Ser Val Ala Ser Ser Ala Ala
            340                 345                 350

Ala Thr Val Leu Thr Gly Val Phe Gly Asp Asp Tyr Ala Phe Val Asp
        355                 360                 365

Ala Thr Glu Val Pro Tyr Gly Leu Pro Pro Arg Ser Phe Gln Ser Phe
    370                 375                 380

Trp Gln Ala Ala Glu Glu Ala Ala Ile Ser Xaa Leu Tyr Gly Gly Ile
385                 390                 395                 400

His Tyr Arg Pro Ala Ile Glu Leu Gly Val Lys Gln Gly Arg Ala Val
                405                 410                 415

Gly Arg Leu Val Leu Glu Arg Leu Arg Lys Glu His
            420                 425

<210>  18
<211>  1287
<212>  DNA
<213>  artificial

<220>
<223>  consensus sequence of ZgvIPO2 functional variant with a histidine
       tag

<220>
<221>  misc_feature
<222>  (742)..(744)
<223>  n is a, c, g, or t

<220>
<221>  misc_feature
<222>  (751)..(753)
<223>  n is a, c, g, or t

<220>
<221>  misc_feature
<222>  (772)..(774)
<223>  n is a, c, g, or t

<220>
<221>  misc_feature
<222>  (913)..(921)
<223>  n is a, c, g, or t

<220>
<221>  misc_feature
<222>  (925)..(927)
<223>  n is a, c, g, or t

<220>
<221>  misc_feature
<222>  (946)..(948)
<223>  n is a, c, g, or t

<220>
<221>  misc_feature
<222>  (1012)..(1014)
<223>  n is a, c, g, or t

<220>
<221>  misc_feature
<222>  (1027)..(1032)

<223>  n is a, c, g, or t

<220>
<221>  misc_feature
<222>  (1183)..(1185)
<223>  n is a, c, g, or t

<400>  18
atgagaggat cgcatcacca tcaccatcac ggatccgcat gcgatacgta ttttgaaggc      60

ggtttgtcga gatacaaccg ggaactcacc aatgtaatcg tatctgatat ttttacccca     120

ccggtggcca gtaggatcta tgcttattcc aatattgcgg cctacgaagg gatacgcttt     180

atggactcga caaaattgtc cctgtcgtcc cgattgaatg ggttaaaaga gttatcgaca     240

cctgatccgt ccaagaatta ttactatccc ttagtgtcaa ttgtagcgtt tacccaggtg     300

ggaaaatcct tggtttttga tttggacaag gtcgaggcca tcaagaacaa gatgctcggg     360

gaggtcaagg aaataggtat ggaccaagaa gtttatcgga attcaattga tctaggggag     420

accctggcaa gcgaaatctt ggcttgggcg gcaaaagatg ggtatctccg ccgtacggca     480

ttgccccgat attcggtcag tgatgacccg ggccgttgga ggcctacgcc accggactat     540

atggaggcca ttgaaccgca ttggaatacc ttacggccct ttgtgcttga ttcggcagca     600

caatttgatc caggactgcc cacgcctttt gacagtgccg aagactcccc attttataag     660

gaagccatgg aggtctatga aacggtggaa aatctagatg atgataaatt ggaggtggcg     720

aagttttggg actgtaaccc gnnnatatcg nnnaccaagg ggcatgtcat gnnnttccaa     780

caacaaattt cccctggggg ccattggatg catatagctg cacagatact ggaagaacaa     840

aaggtcgacg gggtaaaggc tgccgaaacc atgtcgatgt taggggtggc cctggccgat     900

gcctttatca gtnnnnnnnn ncagnnntat aaaagcagtt tgaccnnncc tgaaacctat     960

atcaataact atattgacca agattgggaa cctatattgc aaacgccggc tnnncctgag    1020

catacgnnnn nncatagtgt ggcgtcaagt gcagcagcta cggtacttac cggggtattc    1080

ggcgatgatt atgcatttgt agatgctacg gaagtaccat acggccttcc tccaagaagc    1140

tttcagtcgt tttggcaagc agcggaagaa gcagcgatca gcnnncttta cggggggatt    1200

cactataggc cggccattga actgggcgtt aaacagggca gggccgtggg ccgattggtt    1260

ctcgagcgat taagaaagga gcattga                                        1287

<210>  19
<211>  414
<212>  PRT
<213>  artificial

<220>
<223>  N261A ZgvIPO2 functional variant

<400>  19

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Ala Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  20
<211>  1242
<212>  DNA
<213>  artificial

<220>
<223>  N261A ZgvIPO2 functional variant

<400>  20
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat 660

gataaattgg aggtggcgaa gttttgggac tgtaacccgg ctatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttgggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 21
<211> 414
<212> PRT
<213> artificial

<220>
<223> N261S ZgvIPO2 functional variant

<400> 21

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Ser Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  22
<211>  1242
<212>  DNA
<213>  artificial

<220>
<223>  N261S ZgvIPO2 functional variant

<400>  22
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga gtatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  23
<211>  414
<212>  PRT
<213>  artificial

<220>
<223>  N261F ZgvIPO2 functional variant

<400>  23

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Phe Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  24
<211>  1242
<212>  DNA
<213>  artificial

<220>
<223>  N261F ZgvIPO2 functional variant

<400>  24
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg 420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccgt ttatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttgggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 25
<211> 414
<212> PRT
<213> artificial

<220>
<223> H264A ZgvIPO2 functional variant

<400> 25

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
                100                 105                 110

Met Asp Gln Glu Val Tyr Arg Asn Ser Ile Asp Leu Gly Glu Thr Leu
            115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
                180                 185                 190

Pro Thr Pro Phe Asp Ser Ala Glu Asp Ser Pro Phe Tyr Lys Glu Ala
            195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser Ala Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
                260                 265                 270

Ala Ala Glu Thr Met Ser Met Leu Gly Val Ala Leu Ala Asp Ala Phe
            275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
                340                 345                 350

Val Asp Ala Thr Glu Val Pro Tyr Gly Leu Pro Pro Arg Ser Phe Gln

355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  26
<211>  1242
<212>  DNA
<213>  artificial

<220>
<223>  H264A ZgvIPO2 functional variant

<400>  26
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcggc caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  27
<211>  414
<212>  PRT
<213>  artificial

<220>
<223>  Y271A ZgvIPO2 functional variant

<400>  27

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu

210                 215                 220

Val Ala Lys Phe Trp Asp Cys Asn Pro Asn Ile Ser His Thr Lys Gly
225                 230                 235                 240

His Val Met Ala Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  28
<211>  1242
<212>  DNA
<213>  artificial

<220>
<223>  Y271A ZgvIPO2 functional variant

<400>  28
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt 240

gtagcgttta cccaggtggg aaaatccttg gtttttgatt tggacaaggt cgaggccatc 300

aagaacaaga tgctcgggga ggtcaaggaa ataggtatgg accaagaagt ttatcggaat 360

tcaattgatc taggggagac cctggcaagc gaaatcttgg cttgggcggc aaaagatggg 420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccga atatatcgca caccaagggg 720

catgtcatgg ctttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttgggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 29
<211> 414
<212> PRT
<213> artificial

<220>
<223> Y271F ZgvIPO2 functional variant

<400> 29

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile

65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Phe Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  30
<211>  1242
<212>  DNA
<213>  artificial

<220>
<223>  Y271F ZgvIPO2 functional variant

<400>  30
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt ttttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac     1080

ggccttcctc caagaagctt tcagtcgttt tggcaagcag cggaagaagc agcgatcagc     1140

aggctttacg gggggattca ctataggccg gccattgaac tgggcgttaa acagggcagg     1200

gccgtgggcc gattggttct cgagcgatta agaaaggagc at                        1242

<210>  31
<211>  414
<212>  PRT
<213>  artificial

<220>
<223>  C318A ZgvIPO2 functional variant

<400>  31

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Ala Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  32
<211>  1242
<212>  DNA
<213>  artificial

<220>
<223>  C318A ZgvIPO2 functional variant

<400>  32
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt gcttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  33
<211>  414
<212>  PRT
<213>  artificial

<220>
<223>  C318S ZgvIPO2 functional variant

<400>  33

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Ser Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  34
<211>  1242
<212>  DNA
<213>  artificial

<220>
<223>  C318S ZgvIPO2 functional variant

<400>  34
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt agttgggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 35
<211> 414
<212> PRT
<213> artificial

<220>
<223> W319R ZgvIPO2 functional variant

<400> 35

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Arg Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210> 36
<211> 1242
<212> DNA
<213> artificial

<220>
<223> W319R ZgvIPO2 functional variant

<400> 36
gatacgtatt ttgaaggcgg tttgtcgaga tacaaccggg aactcaccaa tgtaatcgta 60

tctgatattt ttaccccacc ggtggccagt aggatctatg cttattccaa tattgcggcc 120

tacgaaggga tacgctttat ggactcgaca aaattgtccc tgtcgtcccg attgaatggg 180

ttaaaagagt tatcgacacc tgatccgtcc aagaattatt actatccctt agtgtcaatt 240

gtagcgttta cccaggtggg aaaatccttg gtttttgatt tggacaaggt cgaggccatc 300

aagaacaaga tgctcgggga ggtcaaggaa ataggtatgg accaagaagt ttatcggaat 360

tcaattgatc taggggagac cctggcaagc gaaatcttgg cttgggcggc aaaagatggg 420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccga atatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgtagggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 37
<211> 414
<212> PRT
<213> artificial

<220>
<223> D320K ZgvIPO2 functional variant

<400> 37

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Lys Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  38
<211>  1242
<212>  DNA
<213>  artificial

<220>
<223>  D320K ZgvIPO2 functional variant

<400>  38
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat 660

gataaattgg aggtggcgaa gttttgggac tgtaacccga atatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttggaagc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 39
<211> 414
<212> PRT
<213> artificial

<220>
<223> D320Y ZgvIPO2 functional variant

<400> 39

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Tyr Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg

385                 390                 395                 400

Ala Val Gly Arg Leu Val Leu Glu Arg Leu Arg Lys Glu His
                405                 410

<210>  40
<211>  1242
<212>  DNA
<213>  artificial

<220>
<223>  D320Y ZgvIPO2 functional variant

<400>  40
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttggtacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg ggtattcggg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  41
<211>  414
<212>  PRT
<213>  artificial

<220>

<223>  K322R ZgvIPO2 functional variant

<400>  41
   Asp Thr Tyr Phe Glu Gly Gly Leu Ser Arg Tyr Asn Arg Glu Leu Thr
   1               5                   10                  15
   Asn Val Ile Val Ser Asp Ile Phe Thr Pro Pro Val Ala Ser Arg Ile
               20                  25                  30
   Tyr Ala Tyr Ser Asn Ile Ala Ala Tyr Glu Gly Ile Arg Phe Met Asp
           35                  40                  45
   Ser Thr Lys Leu Ser Leu Ser Ser Arg Leu Asn Gly Leu Lys Glu Leu
       50                  55                  60
   Ser Thr Pro Asp Pro Ser Lys Asn Tyr Tyr Tyr Pro Leu Val Ser Ile
   65                  70                  75                  80
   Val Ala Phe Thr Gln Val Gly Lys Ser Leu Val Phe Asp Leu Asp Lys
                   85                  90                  95
   Val Glu Ala Ile Lys Asn Lys Met Leu Gly Glu Val Lys Glu Ile Gly
               100                 105                 110
   Met Asp Gln Glu Val Tyr Arg Asn Ser Ile Asp Leu Gly Glu Thr Leu
           115                 120                 125
   Ala Ser Glu Ile Leu Ala Trp Ala Ala Lys Asp Gly Tyr Leu Arg Arg
       130                 135                 140
   Thr Ala Leu Pro Arg Tyr Ser Val Ser Asp Asp Pro Gly Arg Trp Arg
   145                 150                 155                 160
   Pro Thr Pro Pro Asp Tyr Met Glu Ala Ile Glu Pro His Trp Asn Thr
                   165                 170                 175
   Leu Arg Pro Phe Val Leu Asp Ser Ala Ala Gln Phe Asp Pro Gly Leu
               180                 185                 190
   Pro Thr Pro Phe Asp Ser Ala Glu Asp Ser Pro Phe Tyr Lys Glu Ala
           195                 200                 205
   Met Glu Val Tyr Glu Thr Val Glu Asn Leu Asp Asp Asp Lys Leu Glu
       210                 215                 220
   Val Ala Lys Phe Trp Asp Cys Asn Pro Asn Ile Ser His Thr Lys Gly
   225                 230                 235                 240
   His Val Met Tyr Phe Gln Gln Gln Ile Ser Pro Gly Gly His Trp Met
   245                 250                 255
   His Ile Ala Ala Gln Ile Leu Glu Glu Gln Lys Val Asp Gly Val Lys
               260                 265                 270
   Ala Ala Glu Thr Met Ser Met Leu Gly Val Ala Leu Ala Asp Ala Phe
           275                 280                 285
   Ile Ser Cys Trp Asp Gln Arg Tyr Lys Ser Ser Leu Thr Arg Pro Glu
       290                 295                 300
   Thr Tyr Ile Asn Asn Tyr Ile Asp Gln Asp Trp Glu Pro Ile Leu Gln
   305                 310                 315                 320
   Thr Pro Ala Phe Pro Glu His Thr Ser Gly His Ser Val Ala Ser Ser
                   325                 330                 335
   Ala Ala Ala Thr Val Leu Thr Gly Val Phe Gly Asp Asp Tyr Ala Phe
               340                 345                 350
   Val Asp Ala Thr Glu Val Pro Tyr Gly Leu Pro Pro Arg Ser Phe Gln
           355                 360                 365
   Ser Phe Trp Gln Ala Ala Glu Glu Ala Ala Ile Ser Arg Leu Tyr Gly
       370                 375                 380
   Gly Ile His Tyr Arg Pro Ala Ile Glu Leu Gly Val Lys Gln Gly Arg
   385                 390                 395                 400
   Ala Val Gly Arg Leu Val Leu Glu Arg Leu Arg Lys Glu His
                   405                 410

<210>  42
<211>  1242
<212>  DNA
<213>  artificial

<220>
<223>  K322R ZgvIPO2 functional variant

<400>  42
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccga atatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttgggacc agaggtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 43
<211> 414
<212> PRT
<213> artificial

<220>
<223> R329K ZgvIPO2 functional variant

<400> 43

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly

100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Lys Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  44
<211>  1242
<212>  DNA
<213>  artificial

<220>
<223>  R329K ZgvIPO2 functional variant

<400>  44
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

accaagcctg aaacctatat caataactat attgaccaag attgggaacc tatattgcaa     960

acgccggctt ttcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  45
<211>  414
<212>  PRT
<213>  artificial

<220>
<223>  R329A ZgvIPO2 functional variant

<400>  45

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Ala Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  46
<211>  1242
<212>  DNA
<213>  artificial

<220>
<223>  R329A ZgvIPO2 functional variant

<400>  46
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt 240

gtagcgttta cccaggtggg aaaatccttg gtttttgatt tggacaaggt cgaggccatc 300

aagaacaaga tgctcgggga ggtcaaggaa ataggtatgg accaagaagt ttatcggaat 360

tcaattgatc taggggagac cctggcaagc gaaatcttgg cttgggcggc aaaagatggg 420

tatctccgcc gtacggcatt gccccgatat tcggtcagtg atgacccggg ccgttggagg 480

cctacgccac cggactatat ggaggccatt gaaccgcatt ggaatacctt acggcccttt 540

gtgcttgatt cggcagcaca atttgatcca ggactgccca cgccttttga cagtgccgaa 600

gactccccat tttataagga agccatggag gtctatgaaa cggtggaaaa tctagatgat 660

gataaattgg aggtggcgaa gttttgggac tgtaacccga atatatcgca caccaagggg 720

catgtcatgt atttccaaca acaaatttcc cctgggggcc attggatgca tatagctgca 780

cagatactgg aagaacaaaa ggtcgacggg gtaaaggctg ccgaaaccat gtcgatgtta 840

ggggtggccc tggccgatgc ctttatcagt tgttgggacc agaagtataa aagcagtttg 900

accgcgcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 47
<211> 414
<212> PRT
<213> artificial

<220>
<223> F351H ZgvIPO2 functional variant

<400> 47

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala His Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  48
<211>  1242
<212>  DNA
<213>  artificial

<220>
<223>  F351H ZgvIPO2 functional variant

<400>  48
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctc atcctgagca tacgtctgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc 1140

aggctttacg gggggattca ctataggccg gccattgaac tgggcgttaa acagggcagg 1200

gccgtgggcc gattggttct cgagcgatta agaaaggagc at 1242

<210> 49
<211> 414
<212> PRT
<213> artificial

<220>
<223> S356T ZgvIPO2 functional variant

<400> 49

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Thr Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Arg Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  50
<211>  1242
<212>  DNA
<213>  artificial

<220>
<223>  S356T ZgvIPO2 functional variant

<400>  50
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgactgga catagtgtgg cgtcaagtgc agcagctacg    1020

gtacttaccg gggtattcgg cgatgattat gcatttgtag atgctacgga agtaccatac    1080

ggccttcctc caagaagctt tcagtcgttt tggcaagcag cggaagaagc agcgatcagc    1140

aggctttacg gggggattca ctataggccg gccattgaac tgggcgttaa acagggcagg    1200

gccgtgggcc gattggttct cgagcgatta agaaaggagc at                       1242

<210>  51
<211>  414
<212>  PRT
<213>  artificial

<220>
<223>  R408K ZgvIPO2 functional variant

<400>  51

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Lys Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210>  52
<211>  1242
<212>  DNA
<213>  artificial

<220>
<223>  R408K ZgvIPO2 functional variant

<400>  52
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg 900

acccggcctg aaacctatat caataactat attgaccaag attgggaacc tatattgcaa 960

acgccggctt ttcctgagca tacgtctgga catagtgtgg cgtcaagtgc agcagctacg 1020

gtacttaccg gggtattcgg cgatgattat gcatttgtag atgctacgga agtaccatac 1080

ggccttcctc caagaagctt tcagtcgttt tggcaagcag cggaagaagc agcgatcagc 1140

aagctttacg gggggattca ctataggccg gccattgaac tgggcgttaa acagggcagg 1200

gccgtgggcc gattggttct cgagcgatta agaaaggagc at 1242

<210> 53
<211> 414
<212> PRT
<213> artificial

<220>
<223> R408A ZgvIPO2 functional variant

<400> 53

Asp Thr Tyr Phe Glu Gly Gly Leu Ser Arg Tyr Asn Arg Glu Leu Thr
1               5                   10                  15

Asn Val Ile Val Ser Asp Ile Phe Thr Pro Pro Val Ala Ser Arg Ile
            20                  25                  30

Tyr Ala Tyr Ser Asn Ile Ala Ala Tyr Glu Gly Ile Arg Phe Met Asp
        35                  40                  45

Ser Thr Lys Leu Ser Leu Ser Ser Arg Leu Asn Gly Leu Lys Glu Leu
    50                  55                  60

Ser Thr Pro Asp Pro Ser Lys Asn Tyr Tyr Tyr Pro Leu Val Ser Ile
65                  70                  75                  80

Val Ala Phe Thr Gln Val Gly Lys Ser Leu Val Phe Asp Leu Asp Lys
                85                  90                  95

Val Glu Ala Ile Lys Asn Lys Met Leu Gly Glu Val Lys Glu Ile Gly
            100                 105                 110

Met Asp Gln Glu Val Tyr Arg Asn Ser Ile Asp Leu Gly Glu Thr Leu
        115                 120                 125

Ala Ser Glu Ile Leu Ala Trp Ala Ala Lys Asp Gly Tyr Leu Arg Arg
    130                 135                 140

Thr Ala Leu Pro Arg Tyr Ser Val Ser Asp Asp Pro Gly Arg Trp Arg
145                 150                 155                 160

Pro Thr Pro Pro Asp Tyr Met Glu Ala Ile Glu Pro His Trp Asn Thr
                165                 170                 175

Leu Arg Pro Phe Val Leu Asp Ser Ala Ala Gln Phe Asp Pro Gly Leu
            180                 185                 190

Pro Thr Pro Phe Asp Ser Ala Glu Asp Ser Pro Phe Tyr Lys Glu Ala
        195                 200                 205

Met Glu Val Tyr Glu Thr Val Glu Asn Leu Asp Asp Asp Lys Leu Glu
    210                 215                 220

Val Ala Lys Phe Trp Asp Cys Asn Pro Asn Ile Ser His Thr Lys Gly
225                 230                 235                 240

His Val Met Tyr Phe Gln Gln Gln Ile Ser Pro Gly Gly His Trp Met
                245                 250                 255

His Ile Ala Ala Gln Ile Leu Glu Glu Gln Lys Val Asp Gly Val Lys
            260                 265                 270

Ala Ala Glu Thr Met Ser Met Leu Gly Val Ala Leu Ala Asp Ala Phe
        275                 280                 285

Ile Ser Cys Trp Asp Gln Lys Tyr Lys Ser Ser Leu Thr Arg Pro Glu
    290                 295                 300

Thr Tyr Ile Asn Asn Tyr Ile Asp Gln Asp Trp Glu Pro Ile Leu Gln
305                 310                 315                 320

Thr Pro Ala Phe Pro Glu His Thr Ser Gly His Ser Val Ala Ser Ser
                325                 330                 335

Ala Ala Ala Thr Val Leu Thr Gly Val Phe Gly Asp Asp Tyr Ala Phe
            340                 345                 350

Val Asp Ala Thr Glu Val Pro Tyr Gly Leu Pro Pro Arg Ser Phe Gln
        355                 360                 365

Ser Phe Trp Gln Ala Ala Glu Glu Ala Ala Ile Ser Ala Leu Tyr Gly
    370                 375                 380

Gly Ile His Tyr Arg Pro Ala Ile Glu Leu Gly Val Lys Gln Gly Arg
385                 390                 395                 400

Ala Val Gly Arg Leu Val Leu Glu Arg Leu Arg Lys Glu His
                405                 410

<210> 54

<211>  1242
<212>  DNA
<213>  artificial

<220>
<223>  R408A ZgvIPO2 functional variant

<400>  54
gatacgtatt ttgaaggcgg tttgtcgaga tacaaccggg aactcaccaa tgtaatcgta      60

tctgatattt ttaccccacc ggtggccagt aggatctatg cttattccaa tattgcggcc     120

tacgaaggga tacgctttat ggactcgaca aaattgtccc tgtcgtcccg attgaatggg     180

ttaaaagagt tatcgacacc tgatccgtcc aagaattatt actatccctt agtgtcaatt     240

gtagcgttta cccaggtggg aaaatccttg gtttttgatt tggacaaggt cgaggccatc     300

aagaacaaga tgctcgggga ggtcaaggaa ataggtatgg accaagaagt ttatcggaat     360

tcaattgatc taggggagac cctggcaagc gaaatcttgg cttgggcggc aaaagatggg     420

tatctccgcc gtacggcatt gccccgatat tcggtcagtg atgacccggg ccgttggagg     480

cctacgccac cggactatat ggaggccatt gaaccgcatt ggaatacctt acggcccttt     540

gtgcttgatt cggcagcaca atttgatcca ggactgccca cgccttttga cagtgccgaa     600

gactccccat tttataagga agccatggag gtctatgaaa cggtggaaaa tctagatgat     660

gataaattgg aggtggcgaa gttttgggac tgtaacccga atatatcgca caccaagggg     720

catgtcatgt atttccaaca acaaatttcc cctgggggcc attggatgca tatagctgca     780

cagatactgg aagaacaaaa ggtcgacggg gtaaaggctg ccgaaaccat gtcgatgtta     840

ggggtggccc tggccgatgc ctttatcagt tgttgggacc agaagtataa aagcagtttg     900

acccggcctg aaacctatat caataactat attgaccaag attgggaacc tatattgcaa     960

acgccggctt ttcctgagca tacgtctgga catagtgtgg cgtcaagtgc agcagctacg    1020

gtacttaccg ggtattcggg cgatgattat gcatttgtag atgctacgga agtaccatac    1080

ggccttcctc caagaagctt tcagtcgttt tggcaagcag cggaagaagc agcgatcagc    1140

gcgctttacg gggggattca ctataggccg gccattgaac tgggcgttaa acagggcagg    1200

gccgtgggcc gattggttct cgagcgatta agaaaggagc at                        1242

## Description

The present invention relates to the technical field of halide oxidation during chemical or biochemical processes, using haloperoxidases.

More specifically, the present invention relates to iodide oxidation, using specific and efficient iodoperoxidases from the bacteria *Zobellia galactanivorans,* isolated nucleic acids encoding same, as well as methods for preparing these enzymes.

Moreover, the present invention is directed to the use of such iodoperoxidases in a wide range of industrial, pharmaceutical, medical, cosmetics, and ecological applications, as well as in the food industry.

Halogenated compounds are abundantly found in nature, and play various biological functions when produced by an organism, ranging from chemical defense to signaling molecules. Most organisms capable of incorporating halogens into organic compounds are of marine origin, such as algae and bacteria, and have evolved such capacity thanks to specific enzymes, namely, haloperoxidases.

Haloperoxidases (or HPOs) catalyze, in the presence of hydrogen peroxide, the oxidation of halides according to the following reaction:

H₂O₂ + X⁻ + H⁺ → H₂O + HOX

wherein X⁻ represents a halide ion and is Cl⁻ or Br⁻ or I⁻. A variety of halocarbons can subsequently be generated if the appropriate nucleophilic acceptors are present.

Said haloperoxidases are named according to the most electronegative halide that they can oxidize: chloroperoxidases (CPOs) can catalyze the oxidation of chloride as well as of bromide and iodide, bromoperoxidases (BPOs) react with bromide and iodide, whereas iodoperoxidases (IPOs) are the most specific as they only react with iodide.

Of particular interest is a specific subclass of HPOs that binds a vanadate ion, better known as vanadium-dependant HPOs (or vHPOs): the ability of these enzymes to halogenate a broad range of organic compounds of both commercial and pharmaceutical interest, as well as their high stability towards high temperatures, oxidative conditions and in the presence of organic solvents, make them good candidates for use in industrial biotransformations (Vilter, 1995; Butler et al., 2001).

These properties have elicited detailed structural and mechanistic studies on several vHPOs, namely the CPO from the fungus *Curvularia inaequalis* (Messerschmidt and Wever, 1996), the BPOs from the red algae *Corallina pilulifera* (Shimonishi et al., 1998; Ohshiro et al., 2002) and *Corallina officinalis* (Isupov et al., 2000; Carter et al., 2002) or from the fucalean brown algae *Fucus distichus* (Vreeland et al., 1998) and *Ascophyllum nodosum* (Weyand et al., 1999), as well as the IPOs from *Laminaria digitata* (Colin et al., 2005).

International Patent Application PCT/IB2003/006405, published under N°WO2004/078976, describes new bromo- and iodoperoxidases isolated from the brown algae *Laminaria digitata.* However, WO2004/078976 provides only isolation and subsequent purification of IPOs from the algal sporophytes in order to characterize their biochemical properties and specific activities. Recombinant expression of the IPOs from *L.digitata* has not been demonstrated.

International Patent Application PCT/EP2012/063144 describes a vanadium-dependant iodoperoxidase (vIPO) named *ZgvIPO1* from *Zobellia galactanivorans,* and reports as well for the first time a successful recombinant expression of said iodoperoxidase.

Surprisingly, the present inventors discovered a novel vanadium-dependant haloperoxidase from *Zobellia galactanivorans,* which, despite a low amino acid sequence identity with *ZgvIPO1* (∼48%), is also capable of specifically oxidizing iodide, with similar affinity and catalytic constants (i.e., K_{M} and K_{cat}, respectively). Even more surprisingly, this novel iodoperoxidase shows a higher tolerance to increased concentrations of iodide than *ZgvIPO1.* This advantageous property is particularly desirable in industrial applications which require the processing of highly concentrated iodide.

The present inventors further identified key amino acid residues involved in the biological activity of this novel iodoperoxidase.

The present invention thus provides for the first time a novel iodoperoxidase isolated from *Zobellia* galactanivorans with improved resistance to high concentrations of iodide, functional fragments and functional variants thereof.

More precisely, the present invention provides novel vanadium-dependant iodoperoxidases (vIPOs) of *Zobellia galactanivorans* (ZgvIPOs), said enzymes having highly specific and highly efficient activities for oxidizing iodide.

In the context of the invention, the terms "activity", "function", "biological activity", and "biological function" are equivalent and have to be understood as it is well known in the art. Preferably, such an activity is enzymatic. That is, in the context of the invention, the activity exhibited by the proteins of the invention is one of a iodoperoxidase, such as described above, and can be detected according to the protocol described in paragraph I.4 of the examples below, and/or measured according to the protocol based on the iodination of thymol blue described by Verhaeghe et al. (2008).

A first aspect of the invention relates to an isolated iodoperoxidase (IPO) of *Zobellia galactanivorans,* wherein said iodoperoxidase is selected from a iodoperoxidase comprising at least one sequence selected from the sequences SEQ ID N°1, SEQ ID N°3, SEQ ID N°5, and functional fragments and functional variants thereof.

Preferably, an IPO according to the invention can contain a signal peptide sequence useful for expression of said enzyme in a host cell, such as the putative native signal peptide sequence of *ZgvIPO2* of sequence MKRLSRFIIVCTVLMVSCGTNHDKERL (SEQ ID N°7; such as IPO of sequence SEQ ID N°3), or a protein purification tag, such as a histidine tag (e.g. of sequence SEQ ID N°9: MRGSHHHHHHGSAC; such as IPO of sequence SEQ ID N°5), in the N-terminal extremity and/or C-terminal extremity of said IPO.

Yet preferably, the isolated IPO of *Z.galactanivorans* according to the present invention is selected from a iodoperoxidase consisting of a sequence selected from:
- SEQ ID N°1, wherein a methionin amino-acid can be further present as a translation start signal in the N-terminal extremity of said SEQ ID N°1,
- SEQ ID N°3,
- SEQ ID N°5,
and functional fragments and functional variants thereof.

By "isolated", it is meant free from its natural environment. More precisely, by "isolated", it is meant at least partially purified away from other components. Preferably, "isolated" means that at least one order of magnitude of purification is achieved, preferably two or three orders of magnitude, and most preferably four or five orders of magnitude of purification of the starting material or of the natural material. Thus, the term "isolated" as utilized herein does not necessarily mean that the material of interest is 100% purified and that said material thus excludes any other material.

Preferably, "an isolated iodoperoxidase of *Z*. *galactanivorans"* (ZgIPO) refers to a proteinaceous composition comprising an IPO extracted from *Z. galactanivorans* cells or from recombinant host cells expressing an IPO of *Z. galactanivorans,* wherein said iodoperoxidase is purified to any degree relative to its naturally-obtainable state, i.e., is free from its natural environment. This does not mean in all cases that the isolated ZgIPO is free from any other compounds or impurities, provided the enzyme activity is the only one to be retained by the composition, as may be assessed, for example, by the protein assays described in paragraph I.4 of the examples below, or as would be known to one of ordinary skill in the art for the desired IPO. Substantially pure compositions of at least about 90 to 95% homogeneity are preferred, and 98 to 99% or more homogeneity are yet preferred.

Methods for obtaining said iodoperoxidase are described below.

Once purified, partially or to homogeneity, the IPO protein from *Z.galactanivorans* may then be used as described below.

The terms "protein", "peptide" and "polypeptide" refer to a sequence of amino acids or residues having no specific length. Thus, peptides, oligopeptides, polypeptides and proteins are encompassed by this definition. Also covered by this definition, are polypeptides having undergone post-translational modifications such as polypeptides having covalent attachment of glycosyl groups, acetyl groups, phosphate groups, lipid groups, and the like.

In other words, in the context of the invention, the terms "protein", "peptide" and "polypeptide" are used interchangeably to refer to an "amino acid sequence". Such sequence is preferably that of an enzyme and, more preferably, that of an IPO, such as defined above.

By "functional fragments" of an amino acid sequence of reference having a biological activity of interest, it is meant parts of this amino acid sequence of reference, said parts comprising at least all the regions essential for exhibiting the biological activity of the amino acid sequence of reference. These parts of sequences can be of various lengths, provided the biological activity of the amino acid sequence of reference is retained by said parts. In the context of the invention, the iodoperoxidase activity of the functional fragments can be detected and/or measured as described above.

The above definition can be applied *mutatis mutandis* to "functional fragments" of a nucleic acid of reference, said nucleic acid of reference encoding a protein having a biological function of interest.

By "functional variants" of an amino acid sequence of reference having a biological activity of interest (as defined above), it is meant proteins that structurally differ from the amino acid sequence of reference but that generally retain all the essential functional characteristics of said amino acid sequence of reference. A variant of a protein may be a naturally-occurring variant or a non-naturally occurring variant. Such non-naturally occurring variants of the reference protein can be made, for example, by mutagenesis techniques on the encoding nucleic acids, such as random mutagenesis or site-directed mutagenesis. More preferably, said non-naturally occurring variants are generated by site-directed mutagenesis.

Structural differences may be limited in such a way that the amino acid sequence of reference and the amino acid sequence of the variant may be closely similar overall, and identical in many regions.

Structural differences may result from conservative or non-conservative amino acid mutations such as substitutions, deletions and/or additions between the amino acid sequence of reference and the variant; variant(s) of which the sequence(s) is mutated can thus be referred herein as "mutant(s)". The only proviso is that, even if some amino acids are substituted, deleted and/or added, the biological activity of the amino acid sequence of reference is retained by the variant, that is to say in the context of the present invention, the variant retains a iodoperoxidase activity, as defined above. The activity of said variant may however differ in its efficiency of iodination compared to the activity of the amino acid sequence of reference. In order to identify mutations which help to retain a biological function of interest, one skilled in the art may refer to well-known amino acids classifications, such as classifications based on mutation matrices (e.g. PAM, BLOSUM) or based on the physical, chemical and structural properties of amino acids (e.g. Taylor, 1986; Livingstone et al., 1993). For example, amino acids can be classified according to the nature and size of their side chains, namely the non-polar and neutral amino acids alanine, glycine, isoleucine, leucine, methionine, phenylalanine, proline and valine; the polar and neutral amino acids asparagine, cysteine, glutamine, serine, threonine, tryptophane, and tyrosine; the acidic and polar amino acids aspartic acid and glutamic acid; the basic and polar amino-acids arginine, histidine and lysine; the small amino acids proline, asparagine, serine, alanine, glycine, threonine, cysteine and valine; and the tiny amino acids alanine, glycine, cysteine and serine. Therefore, some amino acids can be interchanged for one another belonging to the same class, while maintaining the properties provided by the amino acids of the sequence of reference. It is understood that to achieve these changes, the skilled person may use codons (triplets of nucleotides or trinucleotides) that are functionally equivalent, that is to say codons that encode the same amino acids, or biologically equivalent amino acids. Additionally, should the person skilled in the art wish to optimize the expression of the proteins of the invention, it can refer to the referenced database on the website http://www.kazusa.or.jp/codon/, which lists the best use of these codons in various organisms and organelles.

"Functional variants" or "functional mutants" of an amino acid sequence of reference according to the present invention include, but are not limited to, amino acid sequences which are at least 95% identical after alignment to the amino acid sequence of reference having a biological activity of interest. These variants can also have 96%, 97%, 98%, 99%, and 99,999% sequence identity to said amino acid sequence of reference. Identity between amino acid sequences can be determined as described below, such as based on a global homology alignment of said amino acid sequences.

Identity between amino acid or nucleic acid sequences can be determined by comparing a position in each of the sequences which may be aligned for the purposes of comparison. When a position in the compared sequences is occupied by the same amino acid or nucleotide, then the sequences are identical at that position. A degree of identity between amino acid sequences is a function of the number of identical amino acid sequences that are shared between these sequences. A degree of sequence identity between nucleic acids is a function of the number of identical nucleotides at positions shared by these sequences.

To determine the percentage of identity between two amino acid sequences or two nucleic acid sequences, the sequences are aligned for optimal comparison. For example, gaps can be introduced in the sequence of a first amino acid sequence or a first nucleic acid sequence for optimal alignment with the second amino acid sequence or second nucleic acid sequence. The amino acid residues or nucleotides at corresponding amino acid positions or nucleotide positions are then compared. When a position in the first sequence is occupied by the same amino acid residue or nucleotide as the corresponding position in the second sequence, the molecules are identical at that position.

The percentage of identity between the two sequences is a function of the number of identical positions shared by the sequences. Hence % identity = number of identical positions / total number of overlapping positions X 100.

In this comparison, the sequences can be of the same length or may be of different lengths.

Optimal alignment of sequences may be conducted by a global homology alignment (i.e. an alignment of all amino acids or nucleotides of each sequence to be compared), such as by the global homology alignment algorithm of Needleman and Wunsch (1972), by computerized implementations of this algorithm or by visual inspection. The best alignment (i.e., resulting in the highest percentage of identity between the compared sequences) generated by the various methods is selected.

In other words, the percentage of sequence identity is calculated by comparing two optimally aligned sequences, determining the number of positions at which the identical amino acid or nucleotide occurs in both sequences to yield the number of matched positions, dividing the number of matched positions by the total number of positions and multiplying the result by 100 to yield the percentage of sequence identity.

According to a preferred embodiment of the present invention, the functional variant comprises at least one sequence selected from the sequences SEQ ID N°1, SEQ ID N°3 and SEQ ID N°5, said sequences being mutated at at least one amino acid position selected from the amino acid positions:
- 234, 237, 244, 291, 292, 293, 295, 302, 324, 329, 330 and 381 of sequence SEQ ID N°1, and combinations thereof;
- 261, 264, 271, 318, 319, 320, 322, 329, 351, 356, 357 and 408 of sequence SEQ ID N°3, and combinations thereof; and
- 248, 251, 258, 305, 306, 307, 309, 316, 338, 343, 344 and 395 of sequence SEQ ID N°5, and combinations thereof.

More preferably, the functional variant according to the invention comprises at least one sequence selected from the sequences SEQ ID N°1, SEQ ID N°3 and SEQ ID N°5, said sequences being mutated at at least one amino acid position selected from the amino acid positions:
- 234 and 324 of sequence SEQ ID N°1, and combination thereof;
- 261 and 351 of sequence SEQ ID N°3, and combination thereof; and
- 248 and 338 of sequence SEQ ID N°5, and combination thereof.

Functional variants according to the invention, include, without limitation, functional variants comprising at least one sequence selected from the consensus sequences SEQ ID N°13, SEQ ID N°15, SEQ ID N°17, and functional fragments thereof.

Preferably, said functional variants of the invention are selected from functional variants consisting of a sequence selected from the consensus sequences:
- SEQ ID N°13, wherein a methionin amino-acid can be further present as a translation start signal in the N-terminal extremity of said SEQ ID N°13,
- SEQ ID N°15,
- SEQ ID N°17,
and functional fragments thereof.

By "consensus sequence", it is meant herein the sequence of a series of related amino acid sequences that reflects the most common choice of amino acid residues at each position. The above definition can be applied *mutatis mutandis* to a "consensus sequence" of a nucleic acid of reference.

Examples of functional variants according to the invention, include, without limitation, functional variants comprising at least one sequence selected from the sequences SEQ ID N°19, SEQ ID N°21, SEQ ID N°23, SEQ ID N°25, SEQ ID N°27, SEQ ID N°29, SEQ ID N°31, SEQ ID N°33, SEQ ID N°35, SEQ ID N°37, SEQ ID N°39, SEQ ID N°41, SEQ ID N°43, SEQ ID N°45, SEQ ID N°47, SEQ ID N°49, SEQ ID N°51 and SEQ ID N°53, and functional fragments thereof.

Preferably, the sequence selected from the sequences SEQ ID N°19, SEQ ID N°21, SEQ ID N°23, SEQ ID N°25, SEQ ID N°27, SEQ ID N°29, SEQ ID N°31, SEQ ID N°33, SEQ ID N°35, SEQ ID N°37, SEQ ID N°39, SEQ ID N°41, SEQ ID N°43, SEQ ID N°45, SEQ ID N°47, SEQ ID N°49, SEQ ID N°51 and SEQ ID N°53 further comprises:
- a methionin amino acid in the N-terminal extremity of said selected sequence;
- a signal peptide sequence useful for expression of said functional variants in a host cell, such as the putative native signal peptide sequence of *ZgvIPO2* of sequence MKRLSRFIIVCTVLMVSCGTNHDKERL (SEQ ID N°7) in the N-terminal extremity of said selected sequence; and/or
- a protein purification tag, such as a histidine tag (e.g. of sequence SEQ ID N°9: MRGSHHHHHHGSAC) in the N-terminal extremity and/or C-terminal extremity of said selected sequence.

An isolated IPO according to the present invention is preferably encoded by a nucleic acid comprising at least one sequence selected from the sequences SEQ ID N°2, SEQ ID N°4, SEQ ID N°6, functional fragments and functional variants thereof, and complementary sequences thereof.

A nucleic acid according to the invention can contain a nucleotide sequence encoding a signal peptide sequence useful for expression of said enzyme in a host cell, such as the putative native signal peptide sequence of *ZgvIPO2* of sequence ATGAAGCGATTAAGTAGGTTTATTATCGTATGTACGGT GCTTATGGTATCCTGTGGAACAAATCACGACAAAGAGCGGTTA (SEQ ID N°8; such as the IPO sequence SEQ ID N°4), or a nucleotide sequence encoding a protein purification tag, such as a histidine tag (SEQ ID N°10; such as the IPO sequence SEQ ID N°6), in the 5' and/or 3' termini of the selected sequence.

Preferably, the isolated IPO of *Z.galactanivorans* according to the present invention is encoded by a nucleic acid consisting of a sequence selected from:
- SEQ ID N°2, wherein a start codon can be further present in the 5' extremity and a stop codon can be further present in the 3' extremity of said SEQ ID N°2,
- SEQ ID N°4,
- SEQ ID N°6,
and functional fragments and functional variants thereof, and complementary sequences thereof.

Start codons include, but are not limited to, the codons (or trinucleotides, or triplets) ATG, AUG, TTG, UUG, GTG, GUG, CTG and CUG, and can be selected by the person skilled in the art based on the host cell wherein said nucleic acid will be translated. Stop codons include, but are not limited to, the codons TAG, UAG, TAA, UAA, TGA and UGA and can be selected by the person skilled in the art based on the host cell wherein said nucleic acid will be translated.

As used herein, the terms « nucleic acid » or « nucleotide sequence » are used interchangeably, and refer to a precise succession of natural nucleotides (namely, A, T, G, C and U) or non-natural nucleotides, corresponding to a single-stranded or double-stranded DNA such as a cDNA, a genomic DNA, or a plasmidic DNA, and the transcription product of said DNA, such as an RNA. According to the invention, the term "oligonucleotide" preferably refers to a nucleic acid of less than 50 nucleotides in length, while the term "polynucleotide" preferably refers to a nucleic acid of greater than 50 nucleotides in length.

The nucleotide sequences of the present invention may be prepared by any known method including, but not limited to, any synthetic method, any recombinant method, any ex *vivo* generation method and the like, as well as combinations thereof.

As used herein, the term "complementary" means that, for example, each nucleotide of a first nucleic acid sequence is paired with the complementary base of a second nucleic acid sequence whose orientation is reversed. Complementary nucleotides are A and T (or A and U) or C and G.

"Variants" of nucleic acid according to the present invention include, but are not limited to, nucleic acid sequences which are at least 95% identical after alignment to the reference nucleic acid encoding the reference protein. These variants can also have 96%, 97%, 98%, 99%, and 99,999% sequence identity to the nucleic acid encoding the reference protein. Identity between nucleic acid sequences can be determined as defined above.

Nucleotide changes present in a nucleic acid variant may be silent, which means that these changes do not alter the amino acid sequence encoded by the reference nucleic acid.

In the context of the present invention, the nucleic acid variants are "functional", that is to say they encode amino acid sequences having a biological activity of interest as defined above.

Functional variants of nucleic acids according to the invention, include, without limitation, functional variants comprising at least one sequence selected from the consensus sequences SEQ ID N°14, SEQ ID N°16, SEQ ID N°18, and functional fragments and complementary sequences thereof.

Preferably, said functional variants of the invention are selected from functional variants consisting of a sequence selected from the consensus sequences:
- SEQ ID N°14, wherein a start codon can be further present in the 5' termini and a stop codon can be further present in the 3' termini of said SEQ ID N°14,
- SEQ ID N°16,
- SEQ ID N°18,
and functional fragments thereof and complementary sequences thereof.

Examples of functional variants of nucleic acids according to the invention, include, without limitation, functional variants comprising at least one sequence selected from the sequences SEQ ID N°20, SEQ ID N°22, SEQ ID N°24, SEQ ID N°26, SEQ ID N°28, SEQ ID N°30, SEQ ID N°32, SEQ ID N°34, SEQ ID N°36, SEQ ID N°38, SEQ ID N°40, SEQ ID N°42, SEQ ID N°44, SEQ ID N°46, SEQ ID N°48, SEQ ID N°50, SEQ ID N°52 and SEQ ID N°54, and functional fragments thereof and complementary sequences thereof.

Preferably, the sequence selected from the sequences SEQ ID N°20, SEQ ID N°22, SEQ ID N°24, SEQ ID N°26, SEQ ID N°28, SEQ ID N°30, SEQ ID N°32, SEQ ID N°34, SEQ ID N°36, SEQ ID N°38, SEQ ID N°40, SEQ ID N°42, SEQ ID N°44, SEQ ID N°46, SEQ ID N°48, SEQ ID N°50, SEQ ID N°52 and SEQ ID N°54 further comprises:
- a codon encoding a methionin amino acid in the 5' termini of said selected sequence ;
- a nucleotide sequence encoding a signal peptide sequence useful for expression of said functional variants in a host cell, such as the putative native signal peptide sequence of *ZgvIPO2* of sequence ATGAAGCGATTAAGTAGGTTTATTATCGTATGTACGGTGCTTATGGT ATCCTGTGGAACAAATCACGACAAAGAGCGGTTA (SEQ ID N°8) in the 5' termini of said selected sequence; and/ or
- a nucleotide sequence encoding a protein purification tag, such as a histidine tag (SEQ ID N°10) in the 5' and/or 3' termini of said selected sequence.

Also encompassed by the term "variants" of a nucleic acid of reference, are nucleic acids which can hybridize to said nucleic acid of reference. Hybridizing nucleic acids can be useful as probes or primers, for example.

For instance, such hybridizing nucleic acids may be at least 10 nucleotides in length or preferably, at least 17 nucleotides in length. They may also be at least 25 or at least 50 nucleotides in length.

In the context of the present invention, hybridizing nucleic acids will preferably hybridize to the nucleic acid of reference under stringent hybridization conditions. One example of stringent hybridization conditions is where attempted hybridization is carried out at a temperature of from about 35°C to about 65°C using a salt solution which is about 0.9 molar. However, the skilled person will be able to vary such conditions appropriate in order to take into account variables such as probe length, base composition, type of ions present, etc.

Examples of hydridizing nucleic acids include, but are not limited to, the nucleic acids of sequence SEQ ID N°11 and SEQ ID N°12.

The nucleic acids disclosed herein are advantageously comprised in a recombinant vector.

The terms "vector", and "plasmid" herein relate to the same tool which is useful for performing procedures of molecular biology and genetic recombination. Such tool is commonly used and very well known in the art. A nucleic acid of interest can thus be inserted into a vector capable of replication in order to amplify said nucleic acid, or to express the protein encoded by said nucleic acid. These vectors are better known as "cloning vectors" (to amplify a nucleic acid) or "expression vectors" (to express a protein), and are publicly available. Such vectors include, without limitation, plasmid vectors, cosmids, YACS, viral vectors (adenovirus, retrovirus, EBV episome), and phage vectors. In particular, the above-mentioned vectors are said to be recombinant in that they are not found in nature combined to said nucleic acid of interest.

Methods for inserting a nucleic acid into such vectors are known to those skilled in the art. Generally, a nucleic acid is inserted into one or more restriction endonuclease site(s) using appropriate techniques known in the art, e.g. via ligation (see, for example, the techniques described in Sambrook et al., 2001; Ausubel et al., 2011). Nucleotide sequences allowing the transcription of said nucleic acid, the expression and/or purification of the protein encoded by said nucleic acid are preferably also contained in the recombinant vector of the invention. These sequences include, generally and without limitation, at least one sequence selected from one or more signal peptide sequence(s), an origin of replication, one or more gene(s) marker(s) selection, an enhancer element, a promoter, a transcription terminator, and possibly a sequence allowing purification of a protein. The insertion of such sequences in said vector can be done via standard ligation techniques known to those skilled in the art, such as mentioned above. It is additionally known to those skilled in the art that, depending on the nucleotide sequences present in the vector, said vector can replicate in different host cells, and/or the protein encoded by said nucleic acid can be expressed in different host cells.

The recombinant vector is advantageously comprised in a recombinant host cell, such as a prokaryotic or a eukaryotic cell.

As used herein, the terms "host cell", "cell" and "cell line", can be used interchangeably, and refer to a prokaryotic or a eukaryotic cell in which the recombinant vector of the invention can be introduced, such as to amplify the nucleic acid as described above, and/or to express the protein encoded by said nucleic acid. To this end, a host cell may be "transfected" or "transformed" by a process known to those skilled in the art by which said vector is transferred or introduced into the host cell. Examples of such methods include, without limitation, electroporation, lipofection, calcium phosphate transfection, transfection using DEAE dextran, microinjection, and biolistics.

The choice of the host cell can be correlated to the choice of said vector, depending on the selected use, namely the cloning of the nucleic acid or the expression of the protein encoded by said nucleic acid. The skilled person will be able to choose the appropriate host cell among the many cell lines that are publicly available, notably via the American Type Culture Collection (ATCC) (www.ATCC.org).

Examples of prokaryotic cells include, without limitation, bacteria such as Gram-negative bacteria of the genus *Escherichia* (e.g. *E*. *coli* RR1, LE392, B, X1776, W3110, DH5 alpha, JM109, KC8, BL21), *Serratia Pseudomonas, Erwinia Methylobacterium, Rhodobacter, Salmonella* and *Zymomonas,* and Gram positive bacteria of the genus *Corynebacterium, Brevibacterium, Bacillus, Arthrobacter,* and *Streptomyces.*

Examples of eukaryotic cells include, without limitation, cells isolated from fungi, plants, and animals. Such cells notably include, yeasts of the genus *Saccharomyces,* cells of the fungi *Aspergillus, Neurospora, Fusarium* and *Trichoderma,* animal cells such as HEK293 cells, NIH3T3, Jurkat, MEF, Vero, HeLa, CHO, W138, BHK, COS-7, MDCK, C127, Saos, PC12, HKG, and insect cells Sf9, Sf21, Hi Five™ or of *Bombyx mori.* The use of insect cells is particularly described in the manual *"*Baculovirus Expression Vectors: A Laboratory Manual", David R. O'Reilly et al. Oxford University Press, USA (1993).

In a second aspect of the invention, the present invention concerns an isolated nucleic acid encoding a iodoperoxidase of *Z.galactanivorans,* such as defined above.

Preferably, said nucleic acid comprises at least one sequence selected from the sequences SEQ ID N°2, SEQ ID N°4, SEQ ID N°6, functional fragments and functional variants thereof, and complementary sequences thereof.

A nucleic acid according to the invention can contain a nucleotide sequence encoding a signal peptide sequence useful for expression of said enzyme in a host cell, such as the putative native signal peptide sequence of *ZgvIPO2* of sequence ATGAAGCGATTAAGTAGGTTTATTATCGTATGTACGG TGCTTATGGTATCCTGTGGAACAAATCACGACAAAGAGCGGTTA (SEQ ID N°8; such as the IPO sequence SEQ ID N°4), or a nucleotide sequence encoding a protein purification tag, such as a histidine tag (SEQ ID N°10; such as the IPO sequence SEQ ID N°6), in the 5' and/or 3' termini said selected sequence.

Preferably, said nucleic acid consists of a sequence selected from the sequences:
- SEQ ID N°2, wherein a start codon can be further present in the 5' extremity and a stop codon can be further present in the 3' extremity of said SEQ ID N°2,
- SEQ ID N°4,
- SEQ ID N°6,
and functional fragments and functional variants thereof, and complementary sequences thereof.

Definitions of nucleic acids, start and stop codons, have been provided above.

The term "isolated" as used herein means that a nucleic acid has been removed from its original environment in which it is naturally present. Indeed, a nucleic acid, when present in a plant, bacteria or animal in its naturally state, is not considered to be isolated, whereas the same nucleic acid, when separated from the adjacent nucleic acid sequences in which it is naturally inserted in the genome of said plant, bacteria or animal, is considered as being "isolated".

The term "isolated" is not meant to exclude artificial or synthetic mixtures with other compounds, or the presence of impurities which do not interfere with the biological activity and which may be present, for example, due to incomplete purification, addition of stabilizers or mixtures with pharmaceutically acceptable excipients and the like.

Functional variants of said nucleic acids have also been described herein above.

A recombinant vector comprising an isolated nucleic acid encoding a iodoperoxidase of *Z.galactanivorans,* and a recombinant host cell comprising said recombinant vector, both as defined above, are also encompassed as further aspects of the present invention.

According to yet a further aspect, the invention is related to a method for obtaining an isolated iodoperoxidase of *Z.galactanivorans,* such as defined above.

Such method comprises at least the steps of:
a) cloning an isolated nucleic acid as defined above, into a recombinant expression vector ;
b) transforming a host cell with said recombinant expression vector ; and
c) expressing said isolated nucleic acid from the recombinant host cell obtained in step b), so as to obtain said iodoperoxidase.

The recombinant vector used in steps a) and b) and the host cell used in steps b) and c) are as defined above.

In particular, the iodoperoxidase of step c) can be obtained by recovering said enzyme from the host cells if the IPO is expressed intracellularly, and/or from the culture medium in which the host cells are cultured if the IPO is expressed extracellularly.

The iodoperoxidase obtained in step c) can be advantageously purified, in a further step of said method, defined as step d). Preferably, said purification step allows the obtention of a 100%-purified or almost 100%-purified protein.

According to an embodiment of the method of the invention, the host cell as described above is cultured in a suitable culture medium under conditions permitting the expression of the nucleic acid, and thus of the iodoperoxidase. The skilled person in the art may use any conventional method allowing the isolation and/or purification of said enzyme. For example, if the protein was expressed in a dissolved form in host cells, the latter are recovered by centrifugation and suspended in a buffer, then a cell-free extract is obtained by destroying cells through example of an ultrasonic homogenizer. From the supernatant obtained by centrifugation of this extract, a purified sample can be obtained using a conventional method or combination of conventional methods to isolate and purify the protein of the invention. These methods include, without limitation, solvent extraction, salting out with ammonium sulphate, desalting, precipitation with organic solvent, gel filtration, preparative electrophoresis, isoelectric focusing, ultrafiltration, various chromatographic methods such as ion exchange chromatography (anionic, using for example a resin such as diethylaminoethyl (DEAE) Sepharose; or cationic, by using for example a resin such as S-Sepharose (Pharmacia), hydrophobic chromatography (using for example a resin such as butyl sepharose or phenyl sepharose), affinity chromatography using antibodies, adsorption chromatography, chromatofocusing, high performance liquid chromatography (HPLC) and reversed phase HPLC.

Moreover, if a nucleotide sequence allowing purification of the protein, such as a histidine tag, is present in the recombinant vector or in the nucleic acid of the invention, as described above, the protein produced can be recovered by cleavage of said sequence through a specific protease (thrombin, trypsin, protease TEV, etc).

In the case where a chromatographic method is used in step d), one or more substeps can be performed and include, without limitation, the binding of the obtained iodoperoxidase on a solid support, such as a chromatography column, a washing step, and an elution step. Said substeps can be repeated in order to achieve a 100% or almost 100% purification of the isolated protein.

According to another advantageous embodiment, the iodoperoxidase obtained in step d) is solubilized in a further step of said method defined as step e), preferably in a buffer containing NaCl. More preferably, said solubilization step allows the obtention of a fully folded and functional protein.

An example of a method allowing the obtention of an isolated iodoperoxidase of *Z.galactanivorans* is described in paragraphs I.1 to 1.3 of the examples below.

In another aspect, the present invention is directed to uses of an isolated iodoperoxidase of *Z.galactanivorans.*

This enzyme can be used in a number of industrial, pharmaceutical, medical, cosmetics and ecological applications, as well as in the food industry.

Indeed, the IPO of the invention can suitably be used for any purpose to which prior art haloperoxidases have been used, and more specifically wherein iodide is used.

In one embodiment, the IPO according to the present invention is useful for obtaining iodinated organic compounds of interest by iodinating non-iodinated organic compounds.

As used herein, the terms "organic compounds" refer to gaseous, liquid, or solid chemical compounds whose molecules contain carbon.

An example of iodination of non-iodinated organic compounds (RH) is as follows, using the iodoperoxidase of the invention:

I- + H₂O₂ + RH + H⁺ → RI + 2H₂O

wherein RI represents a iodinated organic compound.

More particularly, said iodinated organic compounds of interest include, without limitation, active organic compounds and chemical intermediates used during organic chemical synthesis. Said active organic compounds include, without limitation, desinfectants, nutrients, pesticides, drugs, antibiotics, advantageously plant antibiotics, antioxydants, adhesives, and radiocontrast agents.

Examples of iodinated compounds of interest have been reviewed by La Barre et al. (2010), and include, but are not limited to, phenolic compounds (e.g.mono-,di-,tri-,tetra-iodophloroglucinol, dibromoiodophénol and polymers thereof, as well as iodinated phlorotannins such as iodinated fuhalols, phlorethols, fucols, fucophlorethols, eckols and carmalols), volatile hydrocarbon compounds (e.g. iodoform, iodomethane, diiodomethane, bromoiodomethane, iodoethane, iodopropane, iodobutane, etc), terpenes, amino-acids derivatives (e.g. mono-and diiodotyrosine, which are thyroxine precursors) and fatty acids derivatives (e.g. eiseniaiodides).

More specifically, iodomethane, diiodomethane, iodoform can be used as desinfectants or pesticides. Iodomethane, also known as methyl iodide, can additionally be used as a chemical intermediate during organic chemical synthesis, notably for methylating other compounds such as phenols, carboxylic acids, ammonia and derived amines, and for the industrial-scale production of acetic acid and acetic anhydride.

Examples of radiocontrast agents according to the invention include, but are not limited to, 1,3,5-triiodobenzène and derivatives thereof, such as the ionic agents diatrizoate, metrizoate and ioxaglate, and the non-ionic agents ioversol, iopamidol, iohexol, ioxilan, iopromide and iodixanol.
Such agents can be used for X-Ray imagery, such as fluoroscopy.

Additionally, the IPO of the invention can be used to produce adhesives, useful for example in band-aids, due to the capacity of the enzyme to catalyze oxidative cross-linking of natural organic compounds.

In another embodiment, the above-defined IPO can be used for trapping iodine, preferably during bioremediation such as water treatment.

By "bioremediation", it is meant herein the removal of pollutants via a biological molecule, i.e., an enzyme. Radioactive iodine can be cited as an example of pollutant in the above context.

In yet another embodiment, the IPO of Z. *galactanivorans* can be used for the *in situ* production of antibiotic compounds, for example in antifouling treatment of boats.

The present invention is illustrated, while not being limited, by the following figures.

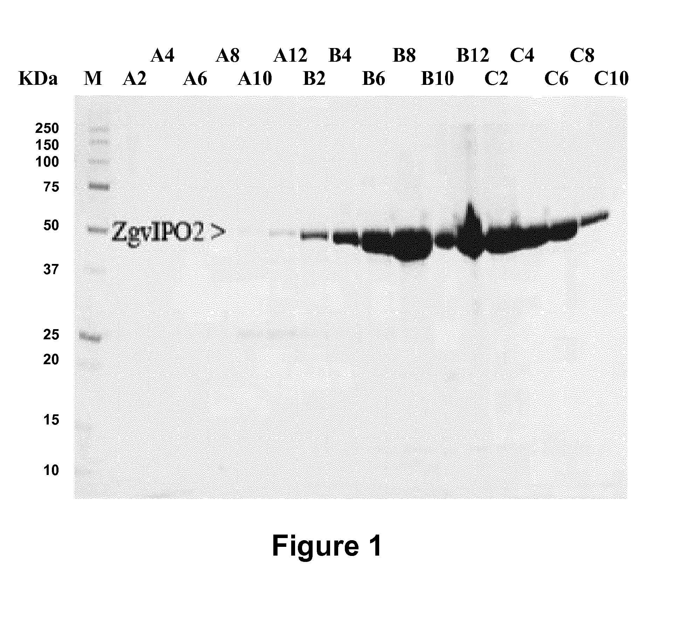

**FIG.1****.** Polyacrylamide gel electrophoresis analysis of the eluted fractions of *ZgvIPO2* obtained after purification by affinity chromatography. The SDS-PAGE gel was stained with Coomassie brilliant blue R-250 to reveal the presence of the eluted proteins. *ZgvIPO2* indicates the presence of the overexpressed iodoperoxidase enzyme. Lanes A2 to C10: eluted protein fractions, M: Precision Plus Protein™ Standards (Bio-Rad Laboratories)

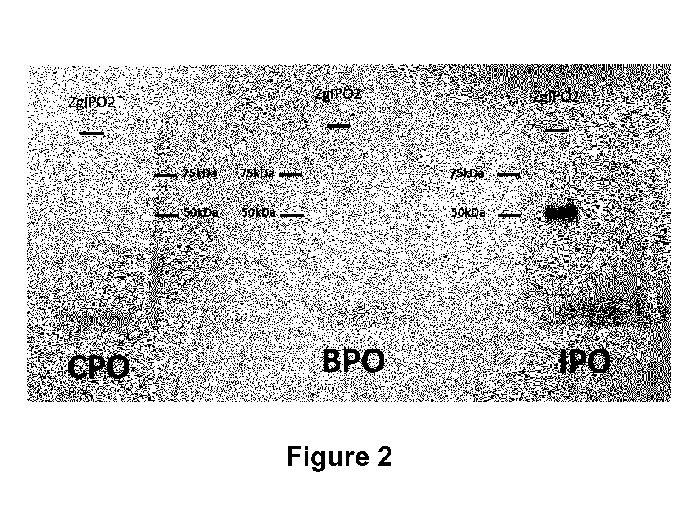

**FIG.2****.** In-gel haloperoxidase assay of the purified recombinant *ZgvIPO2* under non-denaturing PAGE. Polyacrylamide gels were loaded with MilliQ water as a negative control, and with 1 µL of the purified recombinant *ZgvIPO2* enzyme and subsequently stained for chloroperoxidase activity (CPO lanes), bromoperoxidase activity (BPO lanes), and for iodoperoxidase activity (IPO lanes).

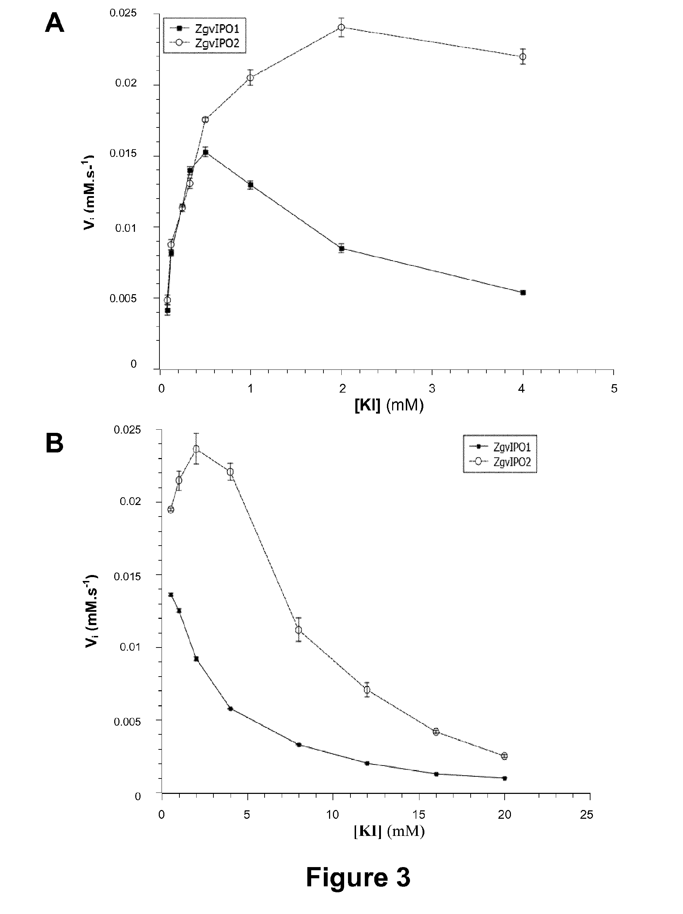

**FIG.3****.** Comparative steady-state analysis of the respective activity of the recombinant vanadium-dependent iodoperoxidases 1 *(ZgvIPO1)* and 2 *(ZgvIPO2)* from *Zobellia galactanivorans* using the Thymol Blue colorimetric assay (Verhaeghe et al. 2008). Mean curves of vᵢ were reported against A) 0.125 mM to 4 mM of KI for the determination of K_{M} and k_{cat} parameters, and B) 0.5 mM to 20 mM of KI for showing the inhibitory effect of a high I⁻ concentration on iodoperoxidase activity.

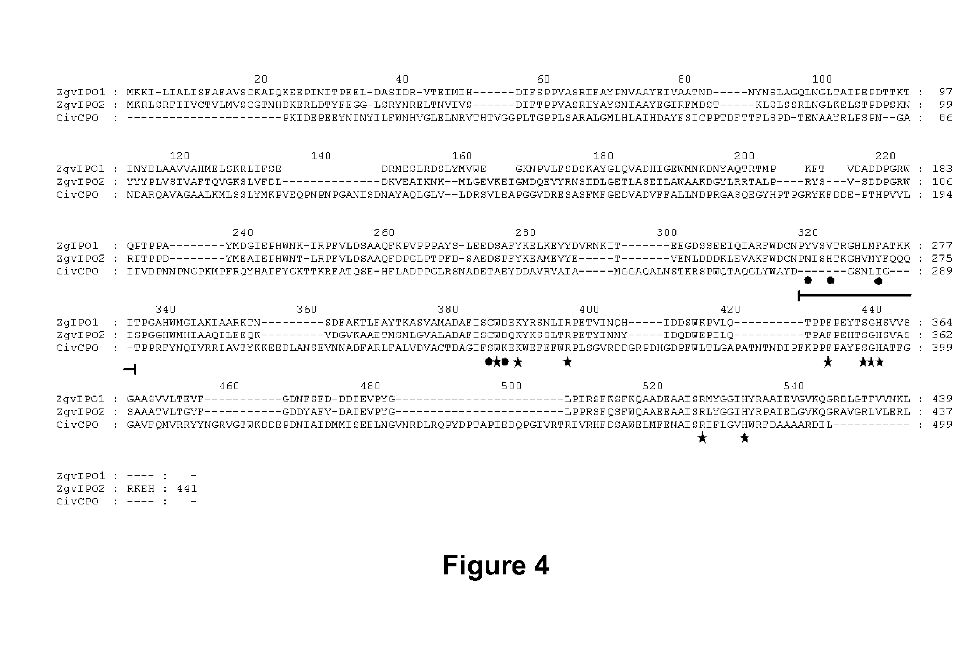

**FIG.4****.** Multiple amino acid sequence alignment of *vIPO1* and *vIPO2* enzymes from *Zobellia galactinovorans* and of the vanadium-chloroperoxidase enzyme of *Curvularia inaequalis* based on three dimensional structure features *(CivCPO,* PDB accession # 1VNC) (protein homology detection by HMM-HMM comparison using the HHpred server).Putative important amino acid residues for catalytic activity are shown with distinctive signs (**-**: supplementary loop in the entrance of the active site; ●: amino acid residues belonging either to the supplementary loop present in the entrance of the active site of *ZgvIPO2,* or to the second coordination sphere of the vanadate cofactor; ★: amino acid residues of the first coordination sphere of the vanadate cofactor).

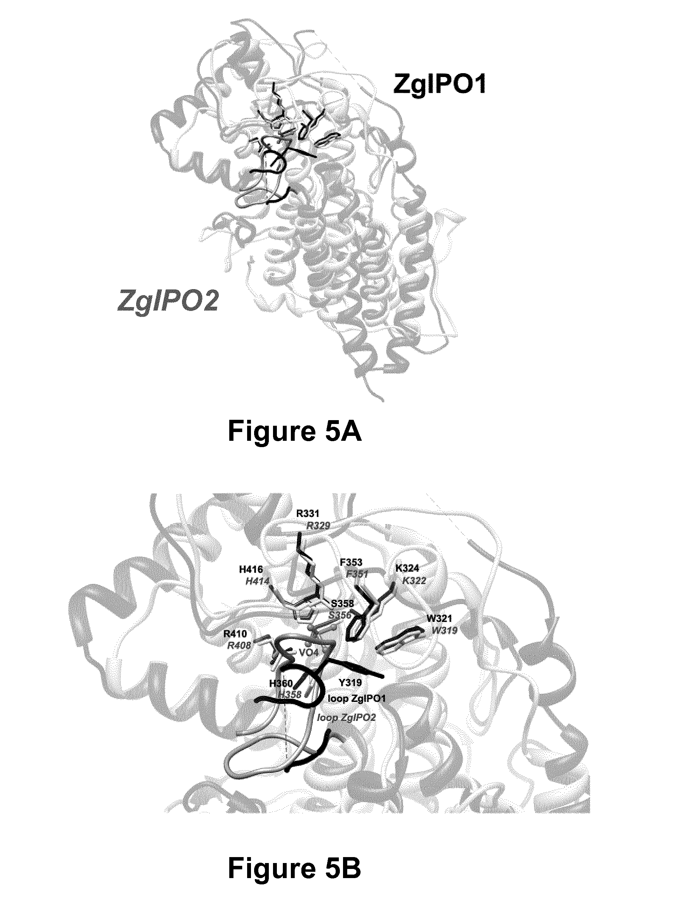

**FIG.5****.** Homology model of the ZgvIPO2 overimposed to the structure of the ZgvIPO1 protein. **5A:** Full view. **5B:** Active site view. *ZgvIPO1* and *ZgvIPO2* secondary structures (alpha-helices, beta-strands and loops) with their active site residues. The additional loops in the *ZgvIPOs* structure are represented in black for *ZgvIPO1* and in light grey for *ZgvIPO2*. The vanadate position is shown by V04.

The present invention will be better understood in the light of the following detailed description of experiments, including examples. Nevertheless, the skilled artisan will appreciate that this detailed description is not limitative and that various modifications, substitutions, omissions, and changes may be made without departing from the scope of the invention.

### EXAMPLES

### I. EXPERIMENTAL PROCEDURES

### I.1. Isolation of bacterial material and genomic DNA

The type strain Dsij^{T} of *Zobellia galactanivorans* was isolated from a red alga (Barbeyron et al. 2001) and grown in ZoBell medium 2216E (ZoBell, 1941) at 20 °C. The genomic DNA was isolated as described by Barbeyron et al. (2001) and fully sequenced.

### I.2. ZgvIPO2 cloning and sequencing

The *Z*. *galactinovorans* genomic DNA was used as a template for polymerase chain reaction (PCR) amplification of the *ZgvIPO2* gene fragment, using the primers of sequence SEQ ID N°11 (forward primer of sequence 5' ACCATCACGGATCCGCATGCGATACGTATTTTGAAGGCGGTTTGTC 3', comprising the *Sph*I restriction site), and SEQ ID N°12 (reverse primer of sequence 5' GCAGGTCGACCCGGGGTACCTCAATGCTCCTTTCTTAATCGC TCG 3', comprising the *Kpn*I restriction site).

The PCR was performed using *Pfu* DNA polymerase, according to the Manufacturer's protocol (Promega) and the PCR product was cloned using a medium throughput cloning program described in Groisillier et al. (2010).

Briefly, the PCR reaction contained 1 µL of *Z. galactinovorans* genomic DNA, 5 µL of each primer (10 µM each), 1 µL dNTPs (10 mM), 5 µL reaction 10x buffer with MgSO₄, 3 µL *Pfu* DNA polymerase, and 35 µL of PCR grade water. The cycling conditions were 96°C for 2 minutes, followed by 35 cycles of 96°C for 15s, 60°C for 30s, and 72°C for 6 minutes, terminating at 72°C for 4 minutes. A 1326 bp PCR amplicon was then digested by the *Sph*I and *Kpn*I restrictions enzymes and cloned into the pQE80L vector (Qiagen) digested by the same restriction enzymes. This expression vector generates a hexa-histidine tail at the N-terminal of recombinant protein, without modifying the recombinant protein activity. This cloning resulted in a gene encoding the *ZgvIPO2* protein, without the first 27 amino-acids coding for the putative signal peptide, flanked by an N-terminal hexa-histidine tag. The pQE80L *ZgvIPO2* vector was first introduced into *Escherichia coli* DH5α, for standard plasmid amplification and sequencing of both strands of the gene was carried out using the ABI Prism 3100 genetic analyzer (Applied Biosystems, CA, USA).

### I.3. Recombinant overexpression of ZgvIPO2 in E. coli and purification

The pQE80L *ZgvIPO2* vector was then transformed into *E.coli* BL21-CodonPlus (DE3)-RIPL (Novagen, Darmstadt, Germany), for overexpression of the enzyme.

Briefly, a 3 mL overnight preculture of a transformed bacteria clone, preselected for its resistance to Ampicillin, was used to inoculate a 500 mL culture of the graffinity medium consisting in a modified LB medium containing 0,5% glucose and 100 µg/mL of Ampicillin. The recombinant bacteria were grown several hours at 37°C until the OD600 reaches 1.4. The culture was then supplemented with 500mL of cold LB medium, 100µg/mL ampicilline, 0.5mM IPTG, 0.6% lactose and 50mM Hepes pH7.5 to induce the production of the *ZgvIPO2* enzyme. The *E.coli* cultured cells were left again for 20h at 20°C for production of *ZgvIPO2,* and then pelleted by centrifugation (at 4000 g for 5 minutes) and frozen at -80°C prior to purification.

The bacterial pellet was suspended in 10mL of lysis buffer (Tris-HCl pH7.5 50mM, 200 mM NaCl) in presence of 1mg/mL of Lysozyme and about 0.25mg/mL of DNase I from bovine pancreas (Sigma Aldrich), and kept on ice during 1h. The suspension was subsequently lysed with a French Press machine. The lysate was then ultra-centrifuged for 1h30min at 30,000 g prior to chromatography separation.

The resulting supernatant was collected and filtered through a 45µm filter mm transferred to a Ni-sepharose column. The extract was fractionated by IMAC affinity using an ÄKTA-purifier (GE Healthcare, USA). After a wash step with buffer A (Tris-HCl pH7.5 50mM; 200mM NaCl; 50mM imidazole), the proteins were eluted using a gradient protocol of 50mM to 500mM imidazole by mixing buffer A and buffer B (Tris-HCl pH7.5 50mM; 200mM NaCl; 500mM imidazole).

A gel electrophoresis of the eluted proteic fractions was carried out using 9% polyacrylamide slab gels according to Laemmli (1970). SDS-samples, containing *β*-mercaptoethanol, were heated at 90°C for 5 min to denature the proteins. The protein samples were then electrophoresed along with a protein molecular mass standard (Precision Plus Protein™ Standards, Bio-Rad Laboratories, Marnes La Coquette, France) and the resulting gel was stained with Coomassie brilliant blue R-250 to reveal the presence of the proteins.

The fractions of interest were then concentrated to a volume of 1mL by ultrafiltration on a CentriPrep Centrifugal Filter Unit 10 kDa (Millipore). The concentrated fraction was dialyzed against 1 liter of final buffer (Tris HCl pH7.5 50mM, 200mM NaCl) using a membrane with a 10 kDa cutoff. The purity of the enzyme was checked by dynamic light scattering.

### I.4. Haloperoxidase activity of the purified ZgvIPO2

Haloperoxidase activities were detected on non-denaturing electrophoresis gels, soaked with 100 mM potassium phosphate buffer, pH 7.4, in the presence of 0.1 mM o-dianisidine, 0.45 mM H₂O₂, and 10 mM potassium iodide, potassium bromide, or potassium chloride in order to reveal iodoperoxidase, bromoperoxidase or chloroperoxidase activities, respectively (Jordan and Vilter, 1990).

Such method allows identifying the specific enzymatic activity of the purified recombinant *ZgvIPO2.*

### I.5. Enzymatic kinetics parameters of the recombinant ZgvIPO2

A spectrophotometric assay to measure the iodoperoxidase activity of the recombinant *ZgvIPO2* was carried out, according to the protocol based on the iodination of Thymol Blue (TB) described by Verhaeghe et al. (Anal. Biochem., 2008).

All reactions were performed in quadruplicate at 20°C in 250 µL, in a 96-wells microplate (Greiner UV-Star 96 well plate, M3812). The stock solution of TB was prepared in H₂O/dimethylsulfoxide (DMSO) (49:1).

The enzymatic kinetics parameters of the recombinant *ZgvIPO2* were directly compared to the recombinant iodoperoxidase *ZgvIPO1* of *Zobellia galactanivorans.* The assays were carried out as follows: to the reaction mixture, consisting of phosphate buffer (110 mM final, pH 7.2), TB (100 µM final), KI (80 µM to 20 mM final) and H₂O₂ (0.35 mM final), was added the purified *ZgvIPO1* and *ZgvIPO2* enzymes and the final concentrations of proteins was 9.1 µg/mL and 8.6 µg/mL, respectively. The iodoperoxidase activity was evaluated by measuring the absorbance of the resulting mixture at 620 nm on a Spectrophotometer SAFIRE II (Tecan, Austria) for 30 min. Each experiment was performed in quadruplicate. A620 values were then converted to millimolars of diiodothymolsulfonphthalein (TBI2) using the equation [C_{TBI2}] = A₆₂₀/ (40.3 mM⁻¹.cm⁻¹ x 0.71 cm).

For the determination of K_{M} and k_{cat} parameters, the experimental initial velocities vᵢ (0 -112 s and [KI] range 0. 125 to 0.5 mM) were expressed in mM of I⁻ converted per minute and the Lineweaver-Burk plots were used to calculate the kinetic parameters K_{M}^{I-} and Vₘₐₓ. k_{cat} values were obtained from k_{cat} = Vₘₐₓ/Eₜ, where Eₜ is the final concentration of *ZgvIPO1* and *ZgvIPO2 (*MW*_{ZgvIPO1}* = 49,404 g.mol⁻¹ and *MW_{ZgvIPO2}* = 50,500 g.mol⁻¹). For full definitions of K_{M}, k_{cat}, Vₘₐₓ.and vᵢ of an enzyme, please see the well-known reference Berg et al. (Biochemistry; 5th edition, 2002).

For thermostability comparison between *ZgvIPOI* and *ZgvIPO2,* recombinant enzymes were analyzed by dynamic light scattering (DLS) measurements from 10 to 65°C. Heating rate was 1°C/4 min. Before measurements, the proteins were centrifuged 1 hour at 13,000 g and 4°C. The sample (120 µL) was transferred to a specific cell. A protein concentration of 1 mg/mL was used. A series of 4 measurements each 1°C was conducted using a Malvern Zetasizer S (Malvern Instruments, UK) with 2 minutes of waiting for a stabilization of the temperature. A green laser (532 nm) was used as the source. The scattered light was collected at a fixed angle of 90° and its output data were processed with the program DTS v4.10 by multiple narrow modes, to obtain hydrodynamic radius (Rh) distributions at each temperature. The melting temperatures *(Tₘ),* corresponding to the temperature where protein start to be denatured, were determined by automatically by the program DTS v4.10.

For the determination of kinetics parameters (Kₘ and k_{cat} values of iodide), the experimental initial velocities (Vᵢ) expressed in millimolars of iodine-converted per minute, were averaged and fitted to the Michaelis-Menten equation.

### I.6. Homology modeling of ZgvIPO2

First, an amino acid alignment of the two protein sequences of *ZgvIPO1* and *ZgvIPO2* with the protein sequence of the vanadium-chloroperoxidase of *Curvularia inaequalis (CivCPO)* was constructed based on structural features of *ZgvIPO1* and *CivCPO,* by using the Hhpred server of the Max Planck Institute (http://toolkit.tuebingen.mpg.de/hhpred). This alignment was used to generate a homology model of *ZgvIPO2* in comparison to the *CivCPO* template on the "Swiss Model" server (http://swissmodel.expasy.org/). The overimposition of this model and the structural coordinates of *ZgvIPO1* was subsequently realized using the program UCSF Chimera v1.6.2. (University of California).

### I.7. ZgvIPO2 mutants

In order to generate various amino acid mutants derived from *ZgvIPO2,* the following mutations as well as combinations thereof are introduced by site-directed mutagenesis into the sequence SEQ ID N°3 of *ZgvIPO2:* N261A, N261 S, N261 F, H264A, Y271A, Y271 F, C318A, C318S, W319R, D320K, D320Y, K322R, R329K, R329A, F351 H, S356T, R408K and R408A.

Each mutant is subsequently cloned for recombinant expression into the pQE80L vector, according to the same procedure as described above.

### II. RESULTS

### II.1. Nucleotide and protein sequence of ZgvIPO2

The annotation of the *ZgvIPO2* gene on the Z. *galactanivorans* genome revealed a 1326 bp sequence (SEQ ID N°4), coding for a protein sequence of 441 amino acids (SEQ ID N°3). The signal peptide was predicted using SIGNALP v.2.0 using both Neural Networks and Hidden Markov models (Nielsen *et al.,* 1999) and was identified as the sequence MKRLSRFIIVCTVLMVSCGTNHDKERL (SEQ ID N°7).

The nucleotide sequence of *ZgvIPO2,* minus the first 81 nucleotides of the 5' end of the sequence coding for the above putative signal peptide, was subsequently cloned in the expression vector pQE80L for production and characterization of the enzyme. The resulting cloned sequence is of sequence SEQ ID N°2 and comprises the stop codon TGA in the 3' extremity of said sequence, and codes for the amino acid sequence SEQ ID N°1. The pQE80L vector carries a hexa-Histidine Tag of sequence SEQ ID N°10 in 5' of sequence SEQ ID N°2 for affinity purification of the protein, without the complete putative signal peptide sequence of the native protein.

### II.2. Overexpression in E.coli and purification of ZgvIPO2

Following overexpression and purification of *ZgvIPO2* in *E*. *coli,* a recombinant protein with the expected size of about 50 kDa was produced, as shown by the gel electrophoresis stained with Coomassie blue of the purified eluted fractions (see Figure 1). A contaminant protein of about 25 kDa was also present in the fractions A10 to B4. As a consequence, only the fractions B6 to C6, devoid of the other contaminant protein, were further concentrated and purified.

The high production yield was estimated to be of about 50 to 100 mg of recombinant protein per 1 L of microbial culture, and can thus support industrial scale production. The protein was fully soluble in presence of 200 mM NaCl, and thus no refolding was necessary.

### II.3. Haloperoxidase activity of ZgvIPO2

The in gel activity assay, under non-denaturing conditions, revealed a strict iodoperoxidase activity of the *ZgvIPO2* enzyme, as shown by Figure 2 where major bands were detected only in the presence of iodide. Neither chloroperoxidase nor bromoperodixase activities were detected.

### II.4. Enzymatic kinetics parameters of the recombinant ZgvIPO2 enzyme

The *ZgvIPO2* specific activity showed typical Michaelis-Menten kinetics as function of iodide in the initial part of the curve, but started to decrease at 4 mM of KI (Figure 3A). Thymol Blue assays were further investigated in presence of KBr but no bromoperoxidase activity was detected for the recombinant *ZgvIPO2* enzyme. The Lineweaver-Burk analysis at fixed levels of iodide from 0.125 to 4 mM revealed alike K_{M}^{I-} of 0.2 mM, and a k_{cat} of 0.96 s⁻¹ for both enzymes (Table 1). However, *ZgvIPO2* showed a stronger resistance to high concentration of KI (up to 4 mM), while *ZgvIPO1* was inhibited above 0.5 mM KI (Figure 3B): this advantageous property might be particularly desirable in industrial applications which require the processing of highly concentrated iodide.

**Table 1. Kinetic parameters using Thymol Blue assay and melting temperature (Tₘ) values determined by dynamic light scattering (DLS) analysis of recombinant purified ZgvIPO1 and ZgvIPO2.**

| | | *Iodide kinetics* | | *DLS analysis* |
|---|---|---|---|---|
| | K_{M}^{I-} | *kcat*^{I-} | *k_{cat}*^{I-}/*K*_{M}^{I-} | *Tₘ* |
| *Enzyme* | (mM) | (s⁻¹) | (s⁻¹.mM⁻¹) | (°C) |
| *ZgvIPO1* | 0.22 ±*0.01* | 1.98±*0.05* | 9.14 | 42 |
| *ZgvIPO2* | 0.22 ±*0.02* | 2.04±*0.08* | 9.32 | 36 |

### II.5. Homology structural model of the ZgvIPO2 enzyme

The *ZgvIPO2* protein was shown to share only 48% of overall amino acid sequence identity with the *ZgvIPO1* protein (Figure 4), which was nevertheless sufficient to generate a homology structural model. The structure of the chloroperoxidase of *Curvularia inaequalis (CivCPO,* PDB accession number # 1VNC) was used as a template to resolve the 3D structures of the *ZgvIPO2* and *ZgvIPO1* enzymes, which were subsequently overimposed on each other (Figure 5A). This modelisation showed that thirteen of the 14 alpha-helices present in *ZgvIPO1* are well-positioned in *ZgvIPO2* model. Similarly, the nine amino acids of the active site appeared to be all conserved both in nature of amino acid residue and in position (Figure 5B, based). An additional loop was also present at the entrance of the catalytic site where the tyrosine Y263 was replaced by an asparagine N261 in *ZgvIPO2* (Figure 5B, based on sequence SEQ ID N°3 of *ZgvIPO2).*

This modelisation further showed that the vanadate binding site of *ZgvIPO2* is situated at the N-terminal of helix 13 at the SGH motif of the sequence SEQ ID N°3. The vanadate cofactor was covalently bond to the Nε2 of His414, a residue conserved in all *vHPO* sequences. Residues of the first coordination sphere of vanadate were shown to be Trp319 and Lys322 in the C-terminal part of helix 11, Arg329, Ser356, Gly357 and His358 in the N-terminal part of helix 13, Phe351 and Arg408 in helix 15 and His414.

### REFERENCES

Vilter, H. (1995) in Metal ions in biological systems (Sigel, H., and Sigel, A., eds) Vol. 31, pp. 325-362, Marcel Dekker, Inc., New York, Basel, Hong Kong.
Butler, A., Carter, J. N., and Simpson, M. T. (2001) in Handbook on Metalloproteins (Bertini, I., Sigel, A., and Sigel, H., eds), pp. 153-179, Marcel Dekker, Inc., New York, Basel.
Messerschmidt, A., and Wever, R. (1996) Proceedings of the National Academy of Sciences of the U.S. 93, 392-396.
Shimonishi, M., Kuwamoto, S., Inoue, H., Wever, R., Ohshiro, T., Izumi, Y., and Tanabe, T. (1998) FEBS letters 428, 105-110.
Ohshiro, T., Hemrika, W., Aibara, T., Wever, R., and Izumi, Y. (2002) Phytochemistry 60, 595-601*.*
Isupov, M. N., Dalby, A. R., Brindley, A. A., Izumi, Y., Tanabe, T., Murshudov, G. N., and Littlechild, J. A. (2000) J. Mol. Biol. 299, 1035-1049
Carter, J. N., Beatty, K. E., Simpson, M. T., and Butler, A. (2002) Journal of Inorganic Biochemistry 91, 59-69.
Vreeland, V., Ng, K. L., and Epstein, L. (1998) Molecular Biology of the Cell 9, 1043.
Weyand, M., Hecht, H., Kiess, M., Liaud, M., Vilter, H., and Schomburg, D. (1999). J Mol Biol. 293(3):595-611.
Colin, C., Leblanc, C., Michel, G., Wagner, E., Leize-Wagner, E., Van Dorsselaer, A. and Potin, P. (2005). J. Biol. Inorg. Chem. 10,156-166.
Verhaeghe, E., Buisson, D., Zekri, E., Leblanc, C., Potin, P., and Ambroise, Y. (2008). Anal Biochem. 379(1):60-65.
Taylor, W.R. (1986). J Theor Biol., 119(2):205-18.
Livingstone, C.D., and Barton, G.J. (1993). Comput. Appl. Biosci., 9(6):745-56
Smith and Waterman (1981) J. Theor. Biol., 91(2), 370-380.
Needleman and Wunsch (1972) J. Mol. Biol. 48(3), 443-453.
Pearson and Lipman (1988) PNAS USA 85(5), 2444-2448.
Altschul, S.F., Gish, W., Miller, W., Myers, E.W., and Lipman, D.J. (1990). J Mol Biol , 215(3), 403-410.
Sambrook et al. (2001). Molecular Cloning: A Laboratory Manual, Cold Spring Harbor Laboratory Press; 3rd edition.
Ausubel et al. (2011). Current Protocols in Molecular Biology, John Wiley & Sons. O'Reilly, D.R. et al. (1993). "Baculovirus Expression Vectors: A Laboratory Manual", Oxford University Press, USA.
La Barre, S., Potin, P., Leblanc, C., and Delage L. (2010). Mar. Drugs 8(4), 988-1010.
Barbeyron, T., L'Haridon, S., Corre, E., Kloareg, B., and Potin, P. (2001). Int J Syst Evol Microbiol., 51(Pt 3):985-97.
ZoBell, C. (1941). J. Mar. Res. 4 : 42-75.
Altschul, S.F., Madden, T.L., Schaffer, A.A., Zhang, J., Zhang, Z., Miller, W., and Lipman, D.J. (1997). Nucleic Acids Res., 25: 3389-3402.
Groisillier, A Herve, C., Jeudy, A., Rebuffet, E., Pluchon, P.F., Chevolot, Y., Flament, D., Geslin, C., Morgado, I.M., Power, D., Branno, M., Moreau, H., Michel, G., Boyen, C. and Czjzek, M. (2010). Cell Factories, 9:45.
Studier (2005). Protein Expr Purif, 41:207-234.
Jordan, P. and Vilter, H. (1990). Electrophoresis, 11(8): 653-655.
Berg, J.M., Tymoczko J.L., and Stryer L.(2002). Biochemistry. 5th edition. New York: W. H. Freeman.
Nielsen, H., Brunak, S., and von Heijne, G. (1999). Protein Eng., 12: 3-9.

## Claims

1. An isolated iodoperoxidase of *Zobellia galactanivorans,* wherein said iodoperoxidase is selected from a iodoperoxidase comprising at least one sequence selected from the sequences SEQ ID N°1, SEQ ID N°3, SEQ ID N°5, and functional fragments and functional variants thereof.

2. The isolated iodoperoxidase according to claim 1, wherein said iodoperoxidase is a functional variant comprising at least one sequence selected from the sequences SEQ ID N°1, SEQ ID N°3, SEQ ID N°5, said sequences being mutated at at least one amino acid position selected from the amino acid positions:
- 234, 237, 244, 291, 292, 293, 295, 302, 324, 329, 330, and 381 of sequence SEQ ID N°1, and combinations thereof;
- 261, 264, 271, 318, 319, 320, 322, 329, 351, 356, 357, and 408 of sequence SEQ ID N°3, and combinations thereof; and
- 248, 251, 258, 305, 306, 307, 309, 316, 338, 343, 344, and 395 of sequence SEQ ID N°5, and combinations thereof.

3. An isolated nucleic acid encoding a iodoperoxidase of *Zobellia galactanivorans* according to claim 1 to 2, wherein said nucleic acid comprises at least one sequence selected from the sequences SEQ ID N°2, SEQ ID N°4, SEQ ID N°6, functional fragments and functional variants thereof, and complementary sequences thereof.

4. A recombinant vector comprising an isolated nucleic acid according to claim 3.

5. The recombinant vector according to claim 4, wherein said vector is a cloning vector or an expressing vector.

6. A recombinant host cell comprising a recombinant vector according to claim 4 or 5.

7. The recombinant host cell according to claim 6, wherein said cell is a prokaryotic cell or a eukaryotic cell.

8. A method for obtaining a iodoperoxidase according to claim 1 or 2, comprising at least the steps of:
a) cloning an isolated nucleic acid according to claim 3, into a recombinant expression vector ;
b) transforming a host cell with said recombinant expression vector ;
c) expressing said isolated nucleic acid from the recombinant host cell obtained in step b), so as to obtain said iodoperoxidase.

9. The method according to claim 8, wherein said method further comprises the step d) of purifying said iodoperoxidase.

10. Use of an isolated iodoperoxidase according to claim 1 or 2 for obtaining iodinated organic compounds of interest by iodinating non-iodinated organic compounds.

11. Use according to claim 10, wherein the iodinated organic compounds of interest are active organic compounds selected from desinfectants, nutrients, pesticides, drugs, antibiotics, advantageously plant antibiotics, antioxydants, adhesives, and radiocontrast agents.

12. Use according to claim 10, wherein the iodinated organic compounds of interest are chemical intermediates used during organic chemical synthesis.

13. Use of an isolated iodoperoxidase according to claim 1 or 2 for trapping iodine, preferably during bioremediation such as water treatment.

14. Use of an isolated iodoperoxidase according to claim 1 or 2 for the *in situ* production of antibiotic compounds.
